(19)

Europäisches
Patentamt
European
Patent Office
Office européen
des brevets

(11)  **EP 4 364 711 A1**

(12)    **EUROPEAN PATENT APPLICATION**
published in accordance with Art. 153(4) EPC

(43) Date of publication:
**08.05.2024   Bulletin 2024/19**

(21) Application number: **22916792.9**

(22) Date of filing: **29.12.2022**

(51) International Patent Classification (IPC):
**A61H 1/02** *(2006.01)*    **B25J 9/00** *(2006.01)*
**A61H 37/00** *(2006.01)*

(52) Cooperative Patent Classification (CPC):
**A63B 21/00181; A63B 21/068; A63B 22/00;**
**A63B 24/0075; A63B 24/0087;** A63B 21/0058;
A63B 21/4009; A63B 21/4011; A63B 69/0028;
A63B 2022/0094; A63B 2024/0093;
A63B 2071/0655; A63B 2071/0663;
A63B 2071/0666; A63B 2071/0677;         (Cont.)

(86) International application number:
**PCT/KR2022/021594**

(87) International publication number:
**WO 2023/128651 (06.07.2023 Gazette 2023/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **29.12.2021   KR 20210191322
19.09.2022   KR 20220117784
01.11.2022   KR 20220143773**

(71) Applicant: **Samsung Electronics Co., Ltd.
Suwon-si, Gyeonggi-do 16677 (KR)**

(72) Inventors:
  • **LEE, Seokjae
    Suwon-si Gyeonggi-do 16677 (KR)**

  • **YU, Jungpil
    Suwon-si Gyeonggi-do 16677 (KR)**
  • **SONG, Sukhoon
    Suwon-si Gyeonggi-do 16677 (KR)**
  • **LIM, Bokman
    Suwon-si Gyeonggi-do 16677 (KR)**
  • **KIM, Kyungrock
    Suwon-si Gyeonggi-do 16677 (KR)**
  • **PARK, Jiyoung
    Suwon-si Gyeonggi-do 16677 (KR)**
  • **LEE, Sangkyung
    Suwon-si Gyeonggi-do 16677 (KR)**
  • **CHO, Younghoon
    Suwon-si Gyeonggi-do 16677 (KR)**

(74) Representative: **Gulde & Partner
Patent- und Rechtsanwaltskanzlei mbB
Wallstraße 58/59
10179 Berlin (DE)**

(54)    **METHOD FOR CONTROLLING WEARABLE DEVICE ON BASIS OF EXERCISE MODE, AND ELECTRONIC DEVICE PERFORMING METHOD**

(57)     An electronic device may determine a target exercise mode of a wearable device, determine a value of a first control parameter based on the target exercise mode, generate sensing data based on raw sensing data obtained from the wearable device and the value of the first control parameter, generate a target state factor corresponding to a target type of the target exercise mode based on the sensing data, determine a value of a second control parameter based on the target exercise mode, determine a torque value corresponding to the target type of the target exercise mode based on the target state factor and the value of the second control parameter, and/or control the wearable device based on the torque value.

FIG. 7

(52) Cooperative Patent Classification (CPC): (Cont.)
A63B 2220/16; A63B 2220/40; A63B 2220/51;
A63B 2220/54; A63B 2220/74; A63B 2220/75;
A63B 2225/20; A63B 2225/50

**Description**

Technical Field

Certain example embodiments relate to a technology for controlling a wearable device.

Background Art

Aging demographics have contributed to a growing number of people who experience inconvenience and pain from reduced muscular strength or aging-induced joint problems. Thus, there is a growing interest in walking assist devices that enable elderly users or patients with reduced muscular strength or joint problems to walk with less effort.

Disclosure of the Invention

Technical Goals

According to an example embodiment, an electronic device may include a communication module, including communication circuitry, configured to exchange data with an external device, and at least one processor configured to control the electronic device. The at least one processor may be configured for performing one or more of the following operations: determining a target exercise mode of a wearable device; determining a value of a first control parameter based on the target exercise mode; generating sensing data based on raw sensing data obtained from the wearable device and a value of the first control parameter; generating a target state factor corresponding to a target type of the target exercise mode based on the sensing data; determining a value of a second control parameter based on the target exercise mode; determining a torque value corresponding to the target type of the target exercise mode based on the target state factor and the value of the second control parameter; and controlling the wearable device based on the torque value.

According to an example embodiment, a method of controlling a wearable device performed by an electronic device may be provided, and the method may include: determining a target exercise mode of the wearable device; determining a value of a first control parameter based on the target exercise mode; generating sensing data based on raw sensing data obtained from the wearable device and a value of the first control parameter; generating a target state factor corresponding to a target type of the target exercise mode based on the sensing data; determining a value of a second control parameter based on the target exercise mode; determining a torque value corresponding to the target type of the target exercise mode based on the target state factor and the value of the second control parameter; and controlling the wearable device based on the torque value.

According to an example embodiment, an electronic device may include a communication module, including communication circuitry, configured to exchange data with an external device, and at least one processor configured to control the electronic device. The at least one processor may be configured for performing the following operations: receiving information on one or more exercise modes from a user; generating an exercise program based on the exercise modes; when a wearable device is controlled based on the exercise program, determining a target exercise mode; determining a value of a first control parameter based on the target exercise mode; generating sensing data based on raw sensing data of the wearable device and a value of the first control parameter; generating a target state factor corresponding to a target type of the target exercise mode based on the sensing data; determining a value of a second control parameter based on the target exercise mode; determining a torque value corresponding to the target type of the target exercise mode based on the target state factor and the value of the second control parameter; and controlling the wearable device based on the torque value.

Brief Description of Drawings

FIG. 1 is a diagram illustrating an example configuration of a system for providing an exercise program to a user according to an example embodiment.
FIG. 2 is a block diagram illustrating an example electronic device in a network environment according to an example embodiment.
FIGS. 3A, 3B, 3C, and 3D are diagrams illustrating an example of a wearable device according to an example embodiment.
FIG. 4 is a diagram illustrating an example of a wearable device communicating with an electronic device according to an example embodiment.

FIGS. 5 and 6 are diagrams illustrating an example of outputting a torque by a wearable device according to an example embodiment.

FIG. 7 is a diagram illustrating an example of determining a torque value for controlling a wearable device according to an example embodiment.

FIG. 8 is a flowchart illustrating an example method of controlling a wearable device based on an exercise mode according to an example embodiment.

FIG. 9 is a flowchart illustrating an example method of generating an exercise program based on exercise modes according to an example embodiment.

FIG. 10 is a diagram illustrating an example method of generating an exercise program according to an example embodiment.

FIG. 11 is a diagram illustrating an example of adjusting an execution time and level of each exercise mode of an exercise program according to an example embodiment.

FIG. 12 is a diagram illustrating an example method of adjusting values of control parameters of an exercise mode according to an example embodiment.

FIG. 13 is a flowchart illustrating an example method of outputting feedback information generated based on sensing data according to an example embodiment.

FIG. 14A is a diagram illustrating an example trajectory of joint angles and an example trajectory of a state factor with respect to the joint angles in a target exercise mode, and an example trajectory of a torque value determined based on the state factor and an assistance mode according to an example embodiment.

FIG. 14B is a diagram illustrating an example trajectory of joint angles measured when a target exercise of FIG. 14A is repeatedly performed and an example trajectory of a state factor with respect to the joint angles, and an example trajectory of a torque value determined based on the state factor and an assistance mode according to an example embodiment.

FIG. 14C is a diagram illustrating an example trajectory of a torque value output by a wearable device and an example trajectory of power, when a target exercise of FIG. 14A is repeatedly performed according to an example embodiment.

FIG. 15A is a diagram illustrating an example trajectory of joint angles in a target exercise mode and an example trajectory of a state factor with respect to the joint angles, and an example trajectory of a torque value determined based on the state factor and a resistance mode according to an example embodiment.

FIG. 15B is a diagram illustrating an example trajectory of joint angles measured when a target exercise of FIG. 15A is repeatedly performed and an example trajectory of a state factor with respect to the joint angles, and an example trajectory of a torque value determined based on the state factor and a resistance mode according to an example embodiment.

FIG. 15C is a diagram illustrating an example trajectory of a torque value output by a wearable device and an example trajectory of power, when a target exercise of FIG. 15A is repeatedly performed according to an example embodiment.

FIG. 16 is a flowchart illustrating an example method by which an electronic device actively determines a target exercise mode according to an example embodiment.

FIGS. 17A, 17B, 17C, 17D, 17E, 17F, and 17G are each a flowchart illustrating an example method of controlling a wearable device based on an exercise mode according to an example embodiment.

## Best Mode for Carrying Out the Invention

Hereinafter, various example embodiments of the present disclosure will be described with reference to the accompanying drawings. However, the example embodiments are not intended to limit the present disclosure to some of the example embodiments, but various changes, modifications, equivalents, and/or alternatives of the example embodiments will be apparent after an understanding of the disclosure.

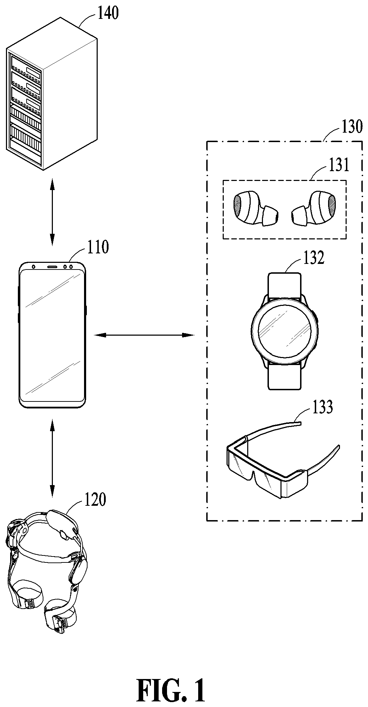

FIG. 1 is a diagram illustrating an example configuration of a system for providing an exercise program to a user according to an example embodiment.

According to an example embodiment, a system for providing an exercise program to a user may include an electronic device 110, a wearable device 120, an additional device 130, and a server 140.

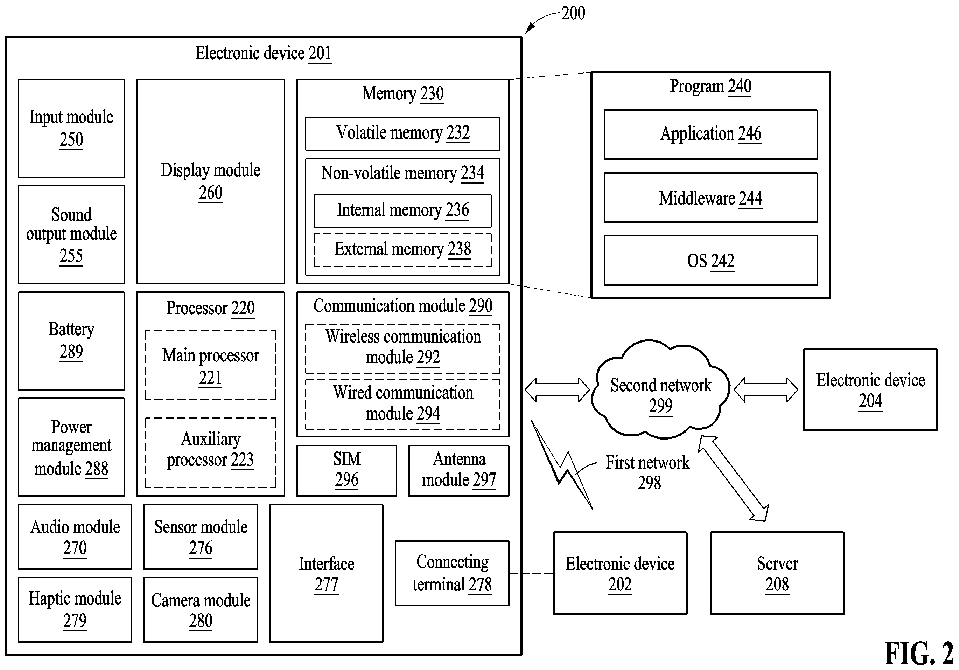

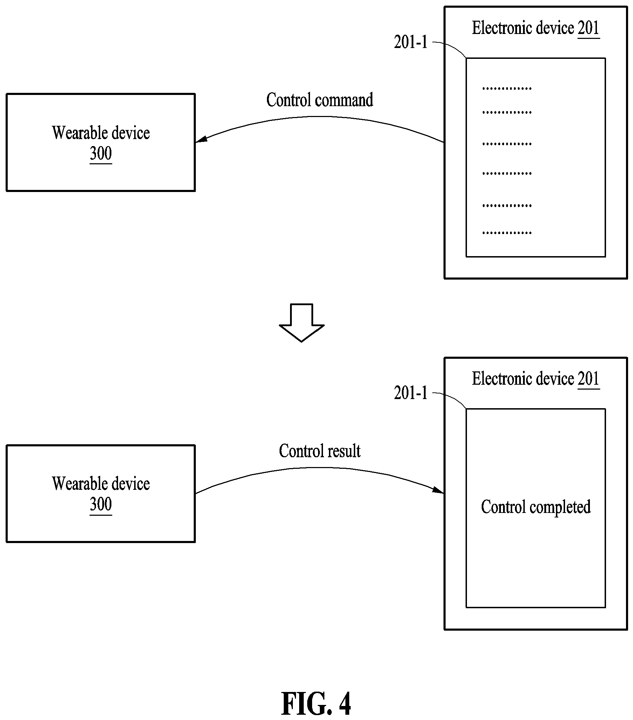

According to an example embodiment, the electronic device 110 may be a user terminal connectable to the wearable device 120 using short-range wireless communication. For example, the electronic device 110 may transmit, to the wearable device 120, a control signal for controlling the wearable device 120. The electronic device 110 will be described in greater detail below with reference to FIG. 2, and the transmission of a control signal will be described in greater detail below with reference to FIG. 4.

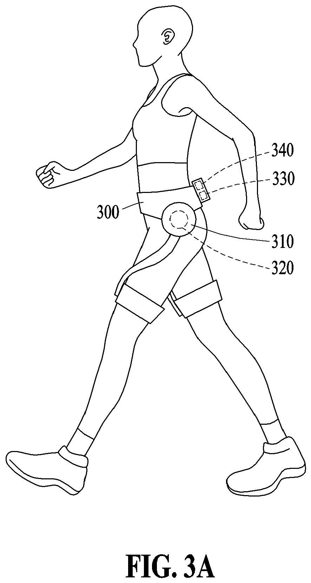

According to an example embodiment, the wearable device 120 may provide a user with an assistance force to assist the user in walking or doing an exercise or with a resistance force to hinder the user from walking. The resistance force may also be provided to the user for the user to do an exercise. The assistance force or the resistance force output by the wearable device 120 may be controlled as values of various control parameters used for the wearable device 120 are controlled. A structure of the wearable device 120 and a method of operating the wearable device 120 will be described in detail below with reference to FIGS. 3A, 3B, 3C, 3D, 4, 5, and 6.

According to an example embodiment, the electronic device 110 may be connected, directly or indirectly, to the additional device 130 (e.g., wireless earphones 131, a smart watch 132, or smart eyeglasses 133) using short-range wireless communication. For example, the electronic device 110 may output information indicating a state of the electronic device 110 or the wearable device 120 to the user through the additional device 130. For example, feedback information on a walking state of the user wearing the wearable device 120 may be output through a haptic device, a speaker device, and a display device of the additional device 130.

According to an example embodiment, the electronic device 110 may be connected, directly or indirectly, to the server 140 using short-range wireless communication or cellular communication.

According to an example embodiment, the server 140 may include a database (DB) in which information on a plurality of exercise modes to be provided to the user through the wearable device 120 is stored.

An exercise mode may refer to a type of exercise to be performed by a user wearing a wearable device. For example, the type of exercise may include a calisthenics type and/or a weights type. For example, the type of exercise may include a new exercise type that is not known. When the user wearing the wearable device 120 performs a motion, an exercise mode may provide a preset force (e.g., torque) corresponding to the motion to the user through the wearable device 120. For example, the force to be provided to the user may be an assistance force to help with the exercise. For example, the force to be provided to the user may be a resistance force to help with the exercise. An output timing and/or a magnitude of the torque to be provided to the user by the wearable device 120 may be controlled as the user changes a previous exercise mode or authors a new exercise mode. An exercise mode may be based on a motion control model that controls the wearable device 120 such that the wearable device 120 provides the user with a suitable torque for a target motion of the user.

The motion control model may be a model (or software, or program) for outputting a torque to the user through the wearable device 120 in a corresponding exercise mode. For example, the motion control model may be a model that controls the wearable device 120 such that a torque corresponding to a motion of the user is output through the wearable device 120. The motion control model may output the torque based on values of control parameters. For example, the control parameters may include parameters for adjusting at least one of a magnitude, direction, and timing of a torque to be output through the wearable device 120, a sensitivity to joint angles of the wearable device 120, or an offset angle between the joint angles.

According to an example embodiment, the server 140 may manage a user account of the user of the electronic device 110 or the wearable device 120. The server 140 may store and manage, in association with the user account, an exercise mode performed by the user and a result of performing the exercise mode.

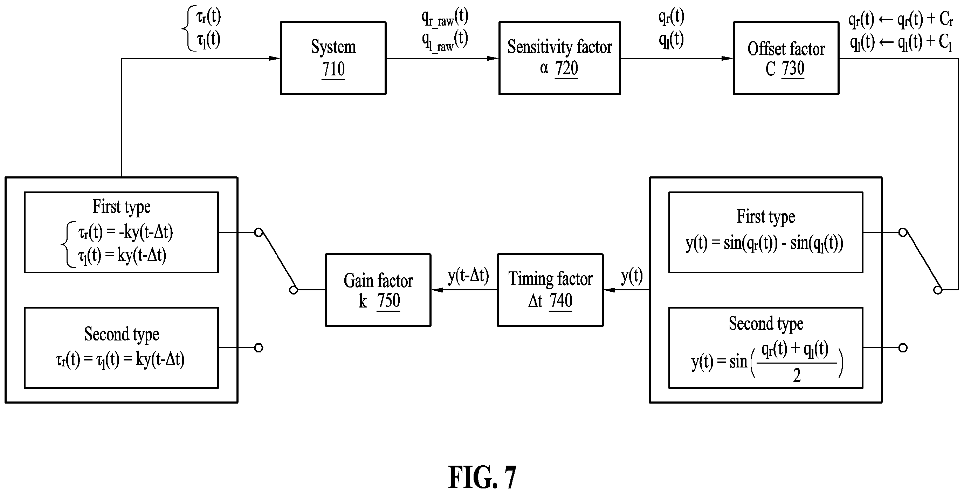

According to an example embodiment, the user wearing the wearable device 120 may perform an exercise mode of a step exercise type or a non-step exercise type. The exercise mode of the step exercise type may include, for example, a walking exercise mode including, for example, a power walking mode, an interval walking mode, a water walking mode, a resisted walking mode, a balance training walking mode, and an assisted walking mode. The exercise mode of the step exercise type may include, for example, a running exercise mode. The exercise mode of the step exercise type may include, for example, an exercise mode in which a left leg and a right leg move symmetrically or alternately, such as, for example, a fast feet mode, a lunge mode, a split jacks mode, a toe-tap triceps mode, a knee up mode, a march step with twist mode, or a mountain climber mode. The exercise mode of the non-step exercise type may include, for example, an exercise mode in which a left leg and a right leg move in the same direction, such as, for example, a squat mode, a narrow squat mode, a half squat mode, a deadlift mode, a single leg deadlift mode, a kick back mode, a bird dog mode, or a good morning mode. The exercise mode of the step exercise type and the exercise mode of the non-step exercise type may apply different methods to output a torque by the wearable device 120, and thus different motion control models may be used therefor. For example, a first motion control model may be applied to a first type which is the step exercise type, and a second motion control model may be applied to a second type which is the non-step exercise type. Based on a motion control model corresponding to an exercise mode, the wearable device 120 may be controlled. A method of providing an exercise mode to a user using the wearable device 120 will be described in detail below with reference to FIGS. 7 through 15C.

FIG. 2 is a block diagram illustrating an example electronic device in a network environment according to an example embodiment.

FIG. 2 is a block diagram illustrating an electronic device 201 (e.g., the electronic device 110 of FIG. 1) in a network environment 200 according to an example embodiment. Referring to FIG. 2, the electronic device 201 in the network environment 200 may communicate with an electronic device 202 via a first network 298 (e.g., a short-range wireless communication network), or communicate with at least one of an electronic device 204 and a server 208 via a second network 299 (e.g., a long-range wireless communication network). According to an example embodiment, the electronic device 201 may communicate with the electronic device 204 via the server 208. According to an example embodiment, the electronic device 201 may include a processor 220, a memory 230, an input module 250, a sound output module 255, a display module 260, an audio module 270, and a sensor module 276, an interface 277, a connecting terminal 278, a haptic module 279, a camera module 280, a power management module 288, a battery 289, a communication module 290, a subscriber identification module (SIM) 296, or an antenna module 297. In an example embodiment, at least one (e.g., the connecting terminal 278) of the above components may be omitted from the electronic device 201, or one or more other components may be added to the electronic device 201. In an example embodiment, some (e.g., the sensor module 276, the camera module 280, or the antenna module 297) of the components may be integrated as a single component (e.g., the display module 260).

The processor 220 may execute, for example, software (e.g., a program 240) to control at least one other component (e.g., a hardware or software component) of the electronic device 201 connected, directly or indirectly, to the processor 220 and may perform various data processing or computations. According to an example embodiment, as at least a part of data processing or computations, the processor 220 may store a command or data received from another component (e.g., the sensor module 276 or the communication module 290) in a volatile memory 232, process the command or data stored in the volatile memory 232, and store resulting data in a non-volatile memory 234. According to an example embodiment, the processor 220 may include a main processor 221 (e.g., a central processing unit (CPU) or an application processor (AP)) or an auxiliary processor 223 (e.g., a graphics processing unit (GPU), a neural processing unit (NPU), an image signal processor (ISP), a sensor hub processor, or a communication processor (CP)) that is operable independently from or in conjunction with, the main processor 221. For example, when the electronic device 201 includes the main processor 221 and the auxiliary processor 223, the auxiliary processor 223 may be adapted to consume less power than the main processor 221 or to be specific to a specified function. The auxiliary processor 223 may be implemented separately from the main processor 221 or as a part of the main processor 221.

The auxiliary processor 223 may control at least some of functions or states related to at least one (e.g., the display module/device 260, the sensor module 276, or the communication module 290) of the components of the electronic device 201, instead of the main processor 221 while the main processor 221 is in an inactive (e.g., sleep) state or along with the main processor 221 while the main processor 221 is an active state (e.g., executing an application). According to an example embodiment, the auxiliary processor 223 (e.g., an ISP or a CP) may be implemented as a portion of another component (e.g., the camera module 280 or the communication module 290) that is functionally related to the auxiliary processor 223. According to an example embodiment, the auxiliary processor 223 (e.g., an NPU) may include a hardware structure specifically for artificial intelligence (AI) model processing. An AI model may be generated by machine learning. The machine learning may be performed by, for example, the electronic device 201, in which the AI model is performed, or performed via a separate server (e.g., the server 208). Learning algorithms may include, but are not limited to, for example, supervised learning, unsupervised learning, semi-supervised learning, or reinforcement learning. The AI model may include a plurality of artificial neural network layers. An artificial neural network may include, for example, a deep neural network (DNN), a convolutional neural network (CNN), a recurrent neural network (RNN), a restricted Boltzmann machine (RBM), a deep belief network (DBN), and a bidirectional recurrent deep neural network (BRDNN), a deep Q-network, or a combination of two or more thereof, but is not limited thereto. The AI model may alternatively or additionally include a software structure other than the hardware structure.

The memory 230 may store various pieces of data used by at least one component (e.g., the processor 220 or the sensor module 276) of the electronic device 201. The various pieces of data may include, for example, software (e.g., the program 240) and input data or output data for a command related thereto. The memory 230 may include the volatile memory 232 or the non-volatile memory 234. The non-volatile memory 234 may include an internal memory 236 and/or an external memory 238.

The program 240 may be stored as software in the memory 230 and may include, for example, an operating system (OS) 242, middleware 244, or an application 246.

The input module 250 may receive, from outside (e.g., a user) the electronic device 201, a command or data to be used by another component (e.g., the processor 220) of the electronic device 201. The input module 250 may include, for example, a microphone, a mouse, a keyboard, a key (e.g., a button), or a digital pen (e.g., a stylus pen).

The sound output module 255 may output a sound signal to the outside of the electronic device 201. The sound output module 255 may include, for example, a speaker or a receiver. The speaker may be used for general purposes, such as playing multimedia or playing a recording. The receiver may be used to receive an incoming call. According to an example embodiment, the receiver may be implemented separately from the speaker or as a part of the speaker.

The display module 260 may visually provide information to the outside (e.g., a user) of the electronic device 201. The display module 260 may include, for example, a display, a hologram device, or a projector, and a control circuitry for controlling a corresponding one of the display, the hologram device, and the projector. According to an example embodiment, the display module 260 may include a touch sensor adapted to sense a touch, or a pressure sensor adapted to measure an intensity of a force of the touch.

The audio module 270 may convert sound into an electric signal or vice versa. According to an example embodiment, the audio module 270 may obtain the sound via the input module 250 or output the sound via the sound output module 255 or an external electronic device (e.g., the electronic device 202, such as a speaker or headphones) directly or wirelessly connected to the electronic device 201.

The sensor module 276 may detect an operational state (e.g., power or temperature) of the electronic device 201 or an environmental state (e.g., a state of a user) external to the electronic device 201 and generate an electric signal or data value corresponding to the detected state. According to an example embodiment, the sensor module 276 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 277 may support one or more specified protocols to be used by the electronic device 201 to couple with an external electronic device (e.g., the electronic device 202) directly (e.g., by wire) or wirelessly. According to an example embodiment, the interface 277 may include, for example, a high-definition multimedia interface (HDMI), a universal serial bus (USB) interface, a secure digital (SD) card interface, or an audio interface.

The connecting terminal 278 may include a connector via which the electronic device 201 may physically connect to an external electronic device (e.g., the electronic device 202). According to an example embodiment, the connecting terminal 278 may include, for example, an HDMI connector, a USB connector, an SD card connector, or an audio connector (e.g., a headphones connector).

The haptic module 279 may convert an electric signal into a mechanical stimulus (e.g., a vibration or a movement) or an electrical stimulus, which may be recognized by a user via their tactile sensation or kinesthetic sensation. According to an example embodiment, the haptic module 279 may include, for example, a motor, a piezoelectric element, or an electric stimulator.

The camera module 280, including at least one camera, may capture a still image and moving images. According to an example embodiment, the camera module 280 may include one or more lenses, image sensors, ISPs, and flashes.

The power management module 288 may manage power supplied to the electronic device 201. According to an example embodiment, the power management module 288 may be implemented as, for example, at least a part of a power management integrated circuit (PMIC).

The battery 289 may supply power to at least one component of the electronic device 201. According to an example embodiment, the battery 289 may include, for example, a primary cell, which is not rechargeable, a secondary cell, which is rechargeable, or a fuel cell.

The communication module 290 may support establishing a direct (e.g., wired) communication channel or a wireless communication channel between the electronic device 201 and an external electronic device (e.g., the electronic device 202, the electronic device 204, or the server 208) and performing communication via the established communication channel. The communication module 290 may include one or more CPs that are operable independently from the processor 220 (e.g., an AP) and that support direct (e.g., wired) communication or wireless communication. According to an example embodiment, the communication module 290 may include a wireless communication module 292 (e.g., a cellular communication module, a short-range wireless communication module, or a global navigation satellite system (GNSS) communication module) or a wired communication module 294 (e.g., a local area network (LAN) communication module or a power line communication (PLC) module). A corresponding one of these communication modules may communicate with the external electronic device, for example, the electronic device 204, via the first network 298 (e.g., a short-range communication network, such as Bluetooth™, wireless-fidelity (Wi-Fi) direct, or infrared data association (IrDA)) or the second network 299 (e.g., a long-range communication network, such as a legacy cellular network, a 5G network, a next-generation communication network, the Internet, or a computer network (e.g., a LAN or a wide area network (WAN)). These various types of communication modules may be implemented as a single component (e.g., a single chip), or may be implemented as multiple components (e.g., multiple chips) separate from each other. The wireless communication module 292 may identify and authenticate the electronic device 201 in a communication network, such as the first network 298 and/or the second network 299, using subscriber information (e.g., international mobile subscriber identity (IMSI)) stored in the SIM 296.

The wireless communication module 292 may support a 5G network after a 4G network and next-generation communication technology (e.g., new radio (NR) access technology). The NR access technology may support enhanced mobile broadband (eMBB), massive machine type communications (mMTC), or ultra-reliable and low-latency communications (URLLC). The wireless communication module 292, including communication circuitry, may support a high-frequency band (e.g., a mmWave band) to achieve, e.g., a high data transmission rate. The wireless communication module 292 may support various technologies for securing performance on a high-frequency band, such as, e.g., beamforming, massive multiple-input and multiple-output (MIMO), full dimensional MIMO (FD-MIMO), an antenna array, analog beamforming, or a large-scale antenna. The wireless communication module 292 may support various requirements specified in the electronic device 201, an external electronic device (e.g., the electronic device 204), or a network system (e.g., the second network 299). According to an example embodiment, the wireless communication module 292 may support a peak data rate (e.g., 20 Gbps or more) for implementing eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 297 may transmit or receive a signal or power to or from the outside (e.g., an external electronic device) of the electronic device 201. According to an example embodiment, the antenna module 297 may include an antenna including a radiating element including a conductive material or a conductive pattern formed in or on a substrate (e.g., a printed circuit board (PCB)). According to an example embodiment, the antenna module 297 may include a plurality of antennas (e.g., an antenna array). In such a case, at least one antenna appropriate for a communication scheme used in a communication network, such as the first network 298 or the second network 299, may be selected by, for example, the communication module 290 from the plurality of antennas. The signal or power may be transmitted or received between the communication module 290 (including communication circuitry) and the external electronic device via the at least one selected antenna. According to an example embodiment, another component (e.g., a radio frequency integrated circuit (RFIC)) other than the radiating element may be additionally formed as a part of the antenna module 297 including at least one antenna.

According to an example embodiment, the antenna module 297 may form a mmWave antenna module. According to an example embodiment, the mmWave antenna module may include a PCB, an RFIC on a first surface (e.g., a bottom surface) of the PCB, or adjacent to the first surface of the PCB and capable of supporting a designated high-frequency band (e.g., a mmWave band), and a plurality of antennas (e.g., an antenna array) disposed on a second surface (e.g., a top or a side surface) of the PCB, or adjacent to the second surface of the PCB and capable of transmitting or receiving signals in the designated high-frequency band.

At least some of the above-described components may be coupled mutually and exchange signals (e.g., commands or data) therebetween via an inter-peripheral communication scheme (e.g., a bus, general-purpose input and output (GPIO), serial peripheral interface (SPI), or mobile industry processor interface (MIPI)).

According to an example embodiment, commands or data may be transmitted or received between the electronic device 201 and the external electronic device (e.g., the electronic device 204) via the server 208 coupled, directly or indirectly, with the second network 299. Each of the external electronic devices (e.g., the electronic device 202 and 204) may be a device of the same type as or a different type from the electronic device 201. According to an example embodiment, all or some of operations to be executed by the electronic device 201 may be executed by one or more of the external electronic devices (e.g., the electronic devices 202 and 204, and the server 208). For example, if the electronic device 201 needs to perform a function or a service automatically, or in response to a request from a user or another device, the electronic device 201, instead of, or in addition to, executing the function or the service, may request one or more external electronic devices to perform at least a part of the function or service. The one or more external electronic devices receiving the request may perform the at least part of the function or service requested, or an additional function or an additional service related to the request, and may transfer a result of the performance to the electronic device 201. The electronic device 201 may provide the result, with or without further processing of the result, as at least a part of a response to the request. To that end, cloud computing, distributed computing, mobile edge computing (MEC), or client-server computing technology may be used, for example. The electronic device 201 may provide ultra-low latency services using, e.g., distributed computing or MEC. In an embodiment, the external electronic device (e.g., the electronic device 204) may include an Internet-of-things (IoT) device. The server 208 may be an intelligent server using machine learning and/or a neural network. According to an example embodiment, the external electronic device (e.g., the electronic device 204) or the server 208 may be included in the second network 299. The electronic device 201 may be applied to intelligent services (e.g., a smart home, a smart city, a smart car, or healthcare) based on 5G communication technology or IoT-related technology.

According to various example embodiments described herein, an electronic device may be a device of one of various types. The electronic device may include, as non-limiting examples, a portable communication device (e.g., a smartphone, etc.), a computing device, a portable multimedia device, a portable medical device, a camera, a wearable device, or a home appliance. However, the electronic device is not limited to the examples described above.

It should be appreciated that various example embodiments of the present disclosure and the terms used therein are not intended to limit the technological features set forth herein to particular embodiments and include various changes, equivalents, or replacements for a corresponding embodiment. In connection with the description of the drawings, like reference numerals may be used for similar or related components. It is to be understood that a singular form of a noun corresponding to an item may include one or more of the things unless the relevant context clearly indicates otherwise. As used herein, "A or B," "at least one of A and B," "at least one of A or B," "A, B, or C," "at least one of A, B, and C," and "A, B, or C," each of which may include any one of the items listed together in the corresponding one of the phrases, or all possible combinations thereof. Terms such as "first," "second," or "initial" or "next" or "subsequent" may simply be used to distinguish the component from other components in question, and do not limit the components in other aspects (e.g., importance or order). It is to be understood that if an element (e.g., a first element) is referred to, with or without the term "operatively" or "communicatively," as "coupled with," "coupled to," "connected with," or "connected to" another element (e.g., a second element), it means that the element may be coupled with the other element directly (e.g., by wire), wirelessly, or via at least a third element.

As used in connection with various example embodiments of the disclosure, the term "module" may include a unit implemented in hardware, software, or firmware, and may interchangeably be used with other terms, for example, "logic," "logic block," "part," or "circuitry." A module may be a single integral component, or a minimum unit or part thereof, adapted to perform one or more functions. For example, according to an example embodiment, the module may be implemented in the form of an application-specific integrated circuit (ASIC). Thus, each "module" herein may comprise circuitry.

Various example embodiments set forth herein may be implemented as software (e.g., the program 240) including one or more instructions that are stored in a storage medium (e.g., the internal memory 236 or the external memory 238) that is readable by a machine (e.g., the electronic device 201). For example, a processor (e.g., the processor 220) of the machine (e.g., the electronic device 201) may invoke at least one of the one or more instructions stored in the storage medium and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to various example embodiments, a method according to an example embodiment of the disclosure may be included and provided in a computer program product. The computer program product may be traded as a product between a seller and a buyer. The computer program product may be distributed in the form of a machine-readable storage medium (e.g., a compact disc read-only memory (CD-ROM)), or be distributed (e.g., downloaded or uploaded) online via an application store (e.g., PlayStore™) or between two user devices (e.g., smart phones) directly. If distributed online, at least part of the computer program product may be temporarily generated or at least temporarily stored in the machine-readable storage medium, such as a memory of the manufacturer's server, a server of the application store, or a relay server.

According to various example embodiments, each component (e.g., a module or a program) of the above-described components may include a single entity or multiple entities, and some of the multiple entities may be separately disposed in different components. According to various example embodiments, one or more of the above-described components or operations may be omitted, or one or more other components or operations may be added. Alternatively or additionally, a plurality of components (e.g., modules or programs) may be integrated into a single component. In such a case, according to various example embodiments, the integrated component may still perform one or more functions of each of the plurality of components in the same or similar manner as they are performed by a corresponding one of the plurality of components before the integration. According to various example embodiments, operations performed by the module, the program, or another component may be carried out sequentially, in parallel, repeatedly, or heuristically, or one or more of the operations may be executed in a different order or omitted, or one or more other operations may be added.

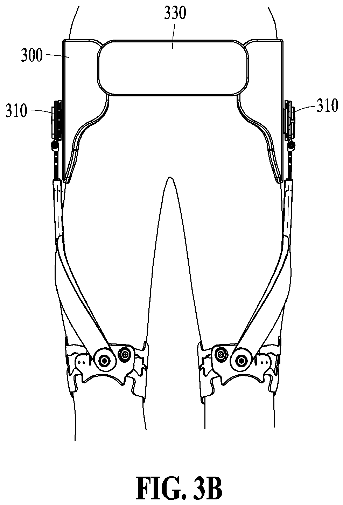

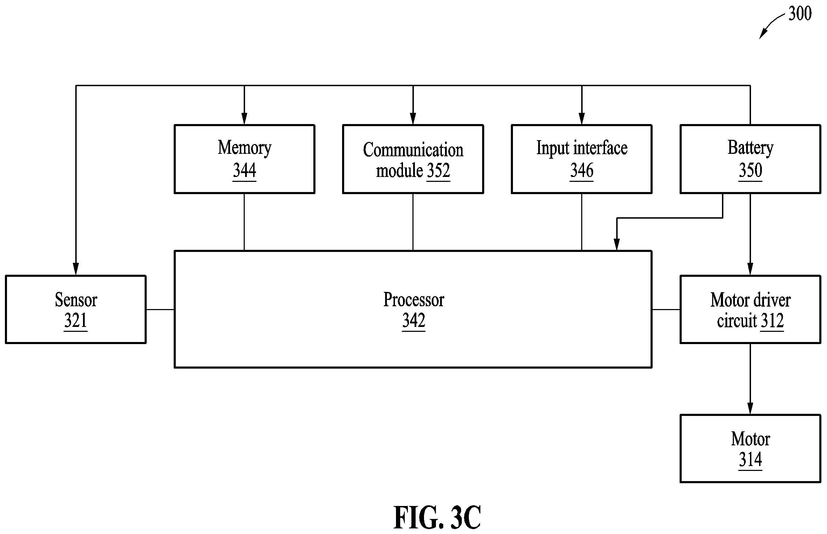

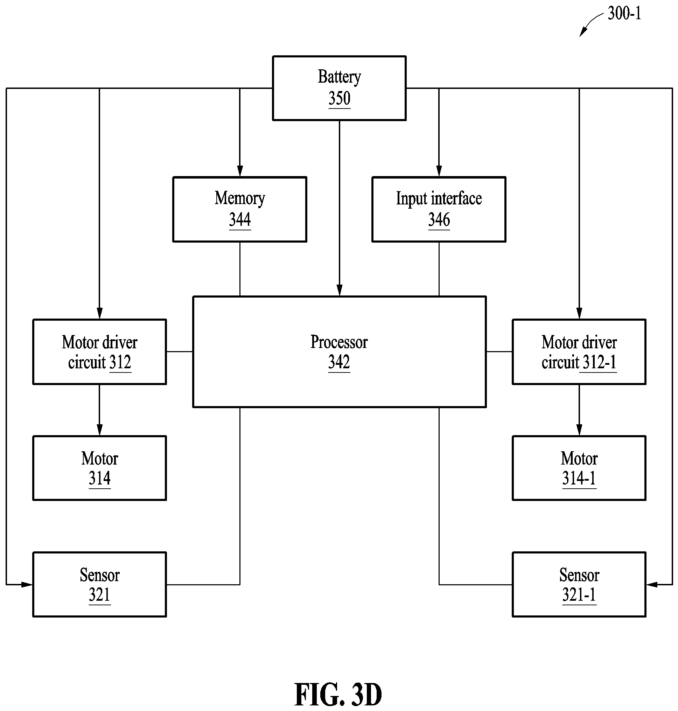

FIGS. 3A, 3B, 3C, and 3D are diagrams illustrating an example of a wearable device according to an example embodiment.

Referring to FIGS. 3A, 3B, 3C, and 3D, a wearable device 300 (e.g., the wearable device 120 of FIG. 1) may be worn on a user to assist the user in walking (and/or gait) more readily. For example, the wearable device 300 may be a device that assists the user in walking. The wearable device 300 may also be an exercise device that not only assists the user with their movements (e.g., walking or doing an exercise) but also provides the user with a resistance force to provide the user with an exercise function. For example, the resistance force provided to the user may be a force that is actively applied to the user, such as, for example, a force output by a device such as a motor. For another example, the resistance force may not be the force that is actively applied to the user but a force that hinders a movement or motion of the user, such as, for example, a frictional force. The resistance force may also be referred to as an exercise load.

Although FIGS. 3A, 3B, 3C, and 3D illustrate an example of a hip-type wearable device, a type of the wearable device 300 is not limited to the illustrated hip type, and the wearable device 300 may be provided in a type that supports a whole lower body, supports a portion of the lower body, for example, a portion of the lower body up to a knee and a portion of the lower body up to an ankle, or supports a whole body.

Although example embodiments described below with reference to FIGS. 3A, 3B, 3C, and 3D apply to a hip-type wearable device, the example embodiments are not limited to the hip-type wearable device but apply to all types of wearable devices.

According to an example embodiment, the wearable device 300 may include a driver 310, a sensor 320, an inertial measurement unit (IMU) 330, a controller 340, a battery 350, and a communication module 352. For example, the IMU 330 and the controller 340 may be arranged in a main frame of the wearable device 300. For another example, the IMU 330 and the controller 340 may be included in a housing formed on or attached to the outside of the main frame of the wearable device 300.

The driver 310 may include a motor 314 and a motor driver circuit 312 for driving the motor 314. The sensor 320 may include at least one sensor 321. The controller 340 may include a processor 342, a memory 344, and an input interface 346. Although the sensor 321, the motor driver circuit 312, and the motor 314 are shown in FIG. 3C as a single sensor, a single motor driver circuit, and a single motor, respectively, another example 300-1 of the wearable device 300 may include a plurality of sensors 321 and 321-1, a plurality of motor driver circuits 312 and 312-1, and a plurality of motors 314 and 314-1 as shown in FIG. 3D. According to implementation, the wearable device 300 may include a plurality of processors. The number of motor driver circuits, the number of motors, or the number of processors may vary according to a body part on which the wearable device 300 is worn.

The following descriptions of the sensor 321, the motor driver circuit 312, and the motor 314 may also apply to the sensor 321-1, the motor driver circuit 312-1, and the motor 314-1 shown in FIG. 3D.

The driver 310 may drive a hip joint of the user. For example, the driver 310 may be disposed at or near a right hip of the user and/or at or near a left hip of the user. The driver 310 may be additionally disposed at or near knees of the user and at or near ankles of the user. The driver 310 may include the motor 314 configured to generate a rotational torque and the motor driver circuit 312 configured to drive the motor 314.

The sensor 320 may measure an angle of the hip joint (hereinafter also be referred to as a hip joint angle) of the user when the user walks. Here, information associated with the hip joint angle sensed by the sensor 320 may include a right hip joint angle, a left hip joint angle, a difference between the right hip joint angle and the left hip joint angle, and a hip joint motion direction. For example, the sensor 321 may be disposed in the driver 310. Based on a position of the sensor 321, the sensor 320 may additionally measure a knee angle of the user and an ankle angle of the user. The sensor 321 may be an encoder. The information associated with the hip joint angle measured by the sensor 320 may be transmitted to the controller 340.

According to an example embodiment, the sensor 320 may include a potentiometer. The potentiometer may sense an R-axis joint angle and an L-axis joint angle, and an R-axis joint angular velocity and an L-axis joint angular velocity, based on a walking motion of the user. In this case, R and L axes may be reference axes for a right leg and a left leg of the user, respectively. For example, the R and L axes may be set to be vertical to the ground and set such that a front side of a body of a person has a negative value and a rear side of the body has a positive value.

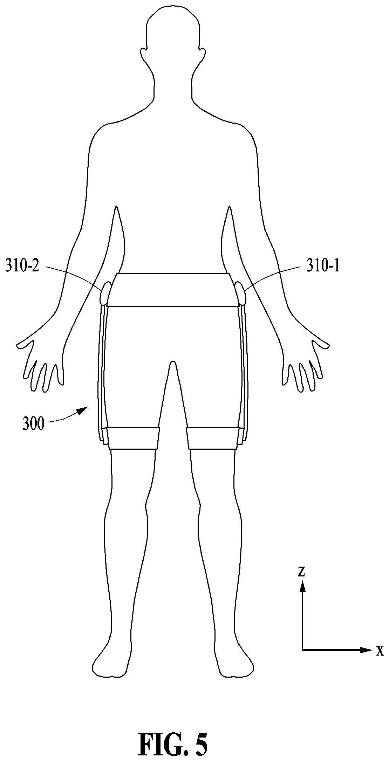

The IMU 330 may measure acceleration information and pose information when the user walks. For example, the IMU 330 may sense an X-axis acceleration, a Y-axis acceleration, and a Z-axis acceleration, and an X-axis angular velocity, a Y-axis angular velocity, and a Z-axis angular velocity, based on a walking motion of the user (e.g., see x, y, and z axes in FIGS. 5-6). The acceleration information and the pose information measured by the IMU 330 may be transmitted to the controller 340.

In addition to the sensor 320 and the IMU 330 described above, the wearable device 300 may include other sensors (e.g., an electromyogram (EMG) sensor) configured to sense a change in a quantity of motion of the user or a change in biosignal based on a walking motion of the user.

The controller 340 may control an overall operation of the wearable device 300. For example, the controller 340 may receive the information sensed by each of the sensor 320 and the IMU 330. The information sensed by the IMU 330 may include the acceleration information and the pose information, and the information sensed by the sensor 320 may include information on the right hip joint angle, the left hip joint angle, the difference between the angles of both hip joints, and the hip joint motion direction. According to an example embodiment, the controller 340 may calculate the difference between the angles of both hip joints based on the right hip joint angle and the left hip joint angle. The controller 340 may generate a signal for controlling the driver 310 based on the sensed information. For example, the generated signal may correspond to an assistance force assisting the user in walking. For another example, the generated signal may correspond to a resistance force hindering the user from walking. The resistance force may also be provided to the user to assist the user in doing an exercise or motion. In the following description, an exercise load (e.g., torque) with a negative magnitude may represent the resistance force, and an exercise load (e.g., torque) with a positive magnitude may represent the assistance force.

According to an example embodiment, the processor 342 of the controller 340 may control the driver 310 to provide the resistance force to the user. For example, the driver 310 may provide the resistance force to the user by applying an active force to the user through the motor 314. For another example, the driver 310 may provide the resistance force to the user using back-drivability of the motor 314 without applying the active force to the user. The back-drivability of a motor may represent the reactivity of a rotation axis of the motor in response to an external force, and a greater degree of the back-drivability may indicate that the motor may more readily respond to an external force acting on the rotation axis of the motor, that is, the rotation axis of the motor may more readily rotate. For example, even when the same external force is applied to the rotation axis of the motor, a degree of rotation of the rotation axis of the motor may change according to a degree of the back-drivability.

According to an example embodiment, the processor 342 of the controller 340 may control the driver 310 such that the driver 310 outputs a torque (and/or an assistance torque) for assisting the user with a movement or motion. For example, in the wearable device 300 of a hip type, the driver 310 may be disposed at or near each of the left hip and the right hip of the user, and the controller 340 may output a control signal for controlling the driver 310 to generate a torque.

The driver 310 may generate the torque based on the control signal output by the controller 340. A torque value for generating the torque may be externally set or be set by the controller 340. For example, to indicate a magnitude of the torque value, the controller 340 may use a magnitude of a current for a signal transmitted to the driver 310. That is, as the magnitude of the current received by the driver 310 increases, the torque value may increase. For example, the processor 342 of the controller 340 may transmit the control signal to the motor driver circuit 312 of the driver 310, and the motor driver circuit 312 may generate a current corresponding to the control signal to control the motor 314.

The battery 350 may supply power to components of the wearable device 300. The wearable device 300 may further include a circuit (e.g., a power management integrated circuit (PMIC)) configured to convert power of the battery 350 to match an operating voltage of the components of the wearable device 300 and provide it to the components of the wearable device 300. In addition, the battery 350 may or may not supply power to the motor 314 based on an operation mode of the wearable device 300.

The communication module 352, including communication circuitry, may support the establishment of a direct (or wired) communication channel or a wireless communication channel between the wearable electronic device 300 and an external electronic device and may support the communication through the established communication channel. The communication module 352 may include one or more communication processors that support direct (or wired) communication or wireless communication. According to an example embodiment, the communication module 352 may include a wireless communication module (e.g., a cellular communication module, a short-range wireless communication module, or a global navigation satellite system (GNSS) communication module) or a wired communication module (e.g., a local area network (LAN) communication module or a power line communication (PLC) module). A corresponding one of these communication modules may communicate with an external electronic device through a first network (e.g., a short-range communication network such as Bluetooth, wireless-fidelity (Wi-Fi) direct, or infrared data association (IrDA)) or a second network (e.g., a legacy cellular network, a 5G network, a next-generation communication network, the Internet, or a computer network). These types of communication modules may be integrated into a single component (e.g., a single chip) or different separate components (e.g., chips).

According to an example embodiment, the electronic device 201 described above with reference to FIG. 2 may be included in the wearable device 300.

According to an example embodiment, the electronic device 201 described above with reference to FIG. 2 may be a device physically separated from the wearable device 300, and the electronic device 201 and the wearable device 300 may be connected through short-range wireless communication.

FIG. 4 is a diagram illustrating an example of a wearable device communicating with an electronic device according to an example embodiment.

Referring to FIG. 4, the wearable device 300 (e.g., the wearable device 120 of FIG. 1) described above with reference to FIGS. 3A, 3B, 3C, and 3D may communicate with the electronic device 201 (e.g., the electronic device 110 of FIG. 1) described above with reference to FIG. 2. For example, the electronic device 201 may be an electronic device of a user of the wearable device 300. The wearable device 300 and the electronic device 201 may be connected through short-range wireless communication.

The electronic device 201 may display a user interface (UI) for controlling operations of the wearable device 300 on a display 201-1. The UI may include, for example, at least one soft key through which the user may control the wearable device 300.

The user may input a command for controlling the operations of the wearable device 300 through the UI on the display 201-1 of the electronic device 201, and the electronic device 201 may generate a control command corresponding to the command and transmit the generated control command to the wearable device 300. The wearable device 300 may operate according to the received control command and transmit a control result to the electronic device 201. The electronic device 201 may display a control completion message on the display 201-1 of the electronic device 201.

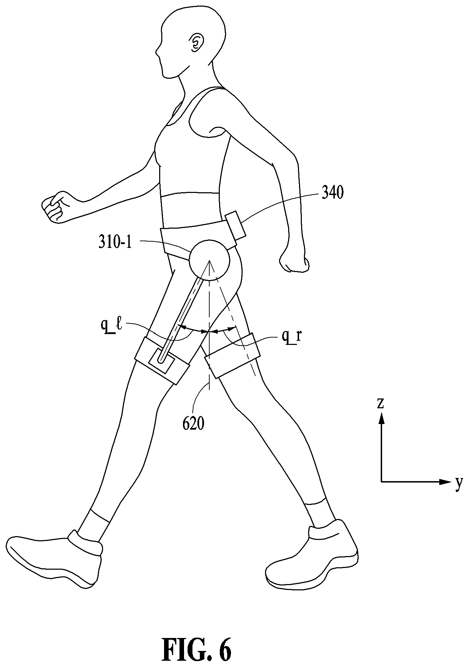

FIGS. 5 and 6 are diagrams illustrating an example of outputting a torque by a wearable device according to an example embodiment.

Referring to FIGS. 5 and 6, drivers 310-1 and 310-2 of the wearable device 300 (e.g., the wearable device 120 of FIG. 1) of FIGS. 3A, 3B, and 3C may be disposed at or near a hip joint of a user, and the controller 340 of the wearable device 300 may be disposed at or near a waist of the user. However, the positions of the drivers 310-1 and 310-2 and the controller 340 are not limited to the example positions shown in FIGS. 5 and 6.

The wearable device 300 may measure (and/or sense) a left hip joint angle $q\_l$ and a right hip joint angle $q\_r$ of the user. For example, the wearable device 300 may measure the left hip joint angle $q\_l$ of the user through a left encoder and measure the right hip joint angle $q\_r$ of the user through a right encoder. As shown in FIG. 6, the left hip joint angle $q\_l$ may be a negative value because a left leg of the user is in front of a reference line 620, and the right hip joint angle $q\_r$ may be a positive value because a right leg of the user is behind the reference line 620. According to implementation, the right hip joint angle $q\_r$ may be negative when the right leg is in front of the reference line 620, and the left hip joint angle q_l may be positive when the left leg is behind the reference line 620.

According to an example embodiment, the wearable device 300 may obtain a first angle (e.g., q_r) and a second angle (e.g., q_l) by filtering a first raw angle (e.g., q_r_raw) of a first joint (e.g., a right hip joint) measured by the sensor 320 and a second raw angle (e.g., q_l_raw) of a second joint (e.g., a left hip joint). For example, the wearable device 300 may filter the first raw angle and the second raw angle based on a previous first angle and a previous second angle that are measured at a previous time. A filtering degree, which is a degree of filtering the raw angles, may be adjusted based on a value of a control parameter indicating a sensitivity of an angle change.

According to an example embodiment, the wearable device 300 may determine a torque value $\tau(t)$ based on a left hip joint angle q_l, a right hip joint angle q_r, an offset angle c, a sensitivity $\alpha$, a gain x, and a delay $\Delta t$, and may control the motor driver circuit 312 of the wearable device 300 such that the determined torque value $\tau(t)$ is output. A force to be provided to the user by the torque value $\tau(t)$ may be referred to herein as force feedback. For example, the wearable device 300 may determine the torque value $\tau(t)$ based on Equation 1 below.

[Equation 1]

$$y = sin(q\_r) - sin(q\_l)$$

$$\tau(t) = \kappa y(t - \Delta t)$$

In Equation 1, y denotes a state factor, and q_r denotes a right hip joint angle, and q_l denotes a left hip joint angle. According to Equation 1, the state factor y may be related to a distance between both legs. For example, y being zero (0) may indicate a state (e.g., a crossing state) in which the distance between the legs is 0, and an absolute value of y being maximum may indicate a state (e.g., a landing state) in which an angle between the legs is maximal. When q_r and q_l are measured at a time t, the state factor may be represented as y(t), in this case.

The gain $\kappa$ is a parameter indicating a magnitude and direction of an output torque. As a magnitude of the gain $\kappa$ increases, a greater torque may be output. When the gain $\kappa$ is a negative value, a torque acting as a resistance force may be output to the user. When the gain $\kappa$ is a positive value, a torque acting as an assistance force may be output to the user. The delay $\Delta t$ is a parameter associated with a torque output timing. The gain $\kappa$ and the delay $\Delta t$ may be preset, and may be adjusted by the user, the wearable device 300, or the electronic device 201 described above with reference to FIG. 2.

A model that outputs the torque acting as the assistance force to the user using Equation 1 may be defined as a torque output model (e.g., a torque output algorithm). The wearable device 300 or the electronic device 201 may input, to the torque output model, values of input parameters received through sensors to determine the magnitude and delay of a torque to be output.

According to an example embodiment, the wearable device 300 or the electronic device 201 may apply, to a first state factor y(t), a first gain value and a first delay value as parameter values determined for the state factor y(t) to determine a first torque value through Equation 2 below.

[Equation 2]

$$\tau_l(t) = \kappa y(t - \Delta t)$$

$$\tau_r(t) = -\kappa y(t - \Delta t)$$

Since it needs be applied to both legs, the calculated first torque value may include a value for the first joint and a value for the second j oint. For example, $\tau_l(t)$ may be a value for the left hip joint which is the second j oint, and $\tau_r(t)$ may be a value for the right hip joint which is the first joint. $\tau_l(t)$ and $\tau_r(t)$ may have the same magnitude and opposite torque directions. The wearable device 300 may control the motor driver circuit 312 of the wearable device 300 such that a torque corresponding to the first torque value is output.

According to an example embodiment, when the user performs a gait in which the left leg and the right leg are asymmetrical, the wearable device 300 may provide an asymmetrical torque to each of the legs of the user to assist such an asymmetric gait. For example, the wearable device 300 may provide a greater (and/or stronger) assistance force to a leg with a smaller stride or a slower swing speed. Hereinafter, a leg with a smaller stride or a slower swing speed will be referred to as an affected leg or a target leg.

In general, the affected leg may have a shorter swing time or a smaller stride compared to an unaffected leg. According to an example embodiment, to assist the user with their gaits, a method of adjusting a timing of a torque acting on the affected leg may be used. For example, to increase an output time of a torque for assisting the affected leg with a swing motion, an offset angle may be added to an actual joint angle of the affected leg. The offset angle may be, for example, an angle that is applied to an actual joint angle measured from the affected leg to maintain a walking balance between the normal leg and the affected leg. As the offset angle increases, the output time of the torque for assisting the affected leg with the swing motion when the user walks may increase. For example, in consideration of a state of the affected leg, an offset angle of -3° to -23° may be determined. As a walking function of the affected leg is more degraded, a greater value of the offset angle may be used. For example, when the affected leg is a right leg of the user, the offset angle may be applied to the right. For example, an angle of a hip joint to which an offset angle of -20° is applied may be changed from a previous angle of -40° to 20° to an angle of -60° to 0°.

As an angle of the affected leg changes by the offset angle, a state factor to be calculated afterward may change. For example, a time for which a positive torque value for assisting the affected leg in moving behind (e.g., a supporting motion) is output may be longer than the normal leg.

c is a value of a parameter indicating an offset angle between joint angles. As the offset angle is added to the actual joint angle of the affected leg, a value of an input parameter to be input to the torque output model provided in (and/ applied to) the wearable device 300 may be adjusted. For example, values of q_r and q_l may be adjusted as represented by Equation 3 below. In addition, $c_r$ denotes an offset angle for the right hip j oint, and $c_l$ denotes an offset angle for the left hip joint.

[Equation 3]

$$q_{-r}(t) \;\leftarrow\; q_{-r}(t) + c_r$$

$$q_{-l}(t) \;\leftarrow\; q_{-l}(t) + c_l$$

According to an example embodiment, the wearable device 300 may filter the state factor to reduce discomfort or inconvenience the user may feel due to an irregular torque output. For example, the wearable device 300 or the electronic device 201 may determine an initial state factor $y_{raw}(t)$ of a present time t based on the first angle of the first joint and the second angle of the second joint, and may determine a first state factor y(t) based on a previous state factor $y^{prv}$ determined at a previous time t-1 and the initial state factor $y_{raw}(t)$. The present time t may indicate a time at which data (e.g., sample) is processed, and the previous time t-1 may indicate a time at which t-1th data is processed. For example, a difference between the present time t and the previous time t-1 may be an operation period of a processor that generates or processes corresponding data. The sensitivity $\alpha$ denotes a value of a parameter indicating sensitivity. For example, a sensitivity value may be continuously adjusted during a test walk, but may be preset as a constant value to reduce the complexity of computation or calculation.

For example, the state factor may be filtered using the sensitivity as represented by Equation 4 below.

[Equation 4]

$$y(t) = (1 - a) \times y(t - 1) + a \times y_{raw}(t)$$

In Equation 4, y(t-1) denotes a previous state factor $y^{prv}$ determined for a previous time t-1. For example, the sensitivity may have a value between 0 and 1. As the sensitivity increases, an influence of an initial state factor $y_{raw}(t)$ may be more greatly applied to a first state factor y(t) determined using Equation 4. For example, in an exercise mode in which a motion of the user changes fast, a greater sensitivity may be set for the wearable device 300 to track fast the fast motion of the user. When a small sensitivity is set in the exercise mode in which the motion of the user changes fast, a speed at which the wearable device 300 tracks the changed motion of the user may decrease.

Although Equation 4 is described that the sensitivity a is applied to the initial state factor $y_{raw}(t)$, it may be implemented in an embodiment in which the sensitivity is applied to y(t-1) or be partially modified and applied to the foregoing embodiment.

According to an example embodiment, a method of outputting a torque based on a state factor described above with reference to Equations 1 to 4 may be used when the user wearing the wearable device 300 walks. For example, when the user performs an in-situ exercise mode (e.g., an exercise mode of a non-step exercise type), instead of a walking mode (e.g., an exercise mode of a step exercise type), a motion control model corresponding to the exercise mode performed by the user may be used to control the wearable device 300.

FIG. 7 is a diagram illustrating an example of determining a torque value for controlling a wearable device according to an example embodiment.

According to an example embodiment, values of control parameters used for a system 710 (e.g., a wearable device (e.g., the wearable device 120 of FIG. 1 or the wearable device 300 of FIGS. 3A, 3B, and 3C) and a user) to output a torque may be determined based on an exercise mode. For example, a target motion control model may determine values of control parameters corresponding to a target exercise mode. The target exercise mode may be an exercise mode currently performed by the user or an exercise mode which is a target to be controlled, and the target motion control model may be a motion control model for the target exercise mode. The target motion control model may control the system 710 based on the values of the control parameters for the target exercise mode. The target motion control model may be executed by the wearable device or an electronic device (e.g., the electronic device 110 of FIG. 1 or the electronic device 201 of FIG. 2).

According to an example embodiment, the target motion control model may determine the values of the control parameters. For example, the control parameters may include at least one of a sensitivity factor 720, an offset factor 730, a timing factor (e.g., delay factor) 740, or a gain factor 750. The values of the control parameters included in the target motion control model may be set differently for each user, and personalization may be performed for each user.

The system 710 may generate a first raw angle (e.g., q_r_raw(t)) of a first joint (e.g., a right hip joint) of the wearable device and a second raw angle (e.g., q_l_raw(t)) of a second joint (e.g., a left hip joint) of the wearable device. A raw angle may be an angle that is obtained through a sensor but is not processed yet, for example, an angle that is not filtered. The target motion control model applied to the system 710 may filter the first raw angle and the second raw joint angle to obtain a first angle (e.g., q_r(t)) and a second angle (e.g., q_l(t)). For example, the target motion control model may filter the first raw angle and the second raw angle based on a previous first angle and a previous second angle that are measured at a previous time. A filtering degree of such raw angle filtering may be adjusted based on a value of the sensitivity factor 720 indicating the sensitivity of an angle change. The target motion control model may be determined by the electronic device (e.g., the electronic device 110 of FIG. 1 or the electronic device 201 of FIG. 2) connected to the wearable device from among a plurality of motion control models.

The target motion control model may adjust the first angle (e.g., q_r(t)) and the second angle (e.g., q_l(t)) using the offset factor 730. As described above, $c_r$ denotes an offset angle with respect to the right hip j oint, and $c_l$ denotes an offset angle with respect to the left hip joint. $c_r$ and $c_l$ may be determined based on a target exercise mode.

The target motion control model may generate a state factor (e.g., y(t)) based on a target type of the target exercise mode. For example, when the target type is a first type (e.g., a step exercise type), the state factor may be generated based on Equation 5 below. For another example, when the target type is a second type (e.g., a non-step exercise type), the state factor may be generated based on Equation 6 below.

[Equation 5]

$$y(t) = \sin\left(q\_r(t)\right) - \sin\left(q\_l(t)\right)$$

[Equation 6]

$$y(t) = \sin\left(\frac{q\_r(t) + q\_l(t)}{2}\right)$$

The state factor generated by Equation 5 may correspond to an exercise mode in which the first joint and the second joint move symmetrically or alternately. The state factor generated by Equation 6 may correspond to an exercise mode in which the first joint and the second joint move in the same direction.

According to an example embodiment, when the wearable device includes a left joint and a right joint, the state factor may be generated using Equation 5 or 6.

According to an example embodiment, when the wearable device includes only one joint, the state factor y(t) may be generated by sin(q(t)) or (q(t)) based on an angle q(t) of the joint.

The target motion control model may determine a value (e.g., Δt) of the timing factor 740 for the state factor (e.g., y(t)). For example, Δt may be determined based on the target exercise mode. Even when users perform the same exercise mode, methods of controlling their bodies may be different for each user, and thus the users may feel different levels of discomfort or inconvenience even though a torque is output at the same timing by the wearable device. Thus, the discomfort or inconvenience at the output timing of the torque may be adjusted through the adjustment of the timing factor 740.

The target motion control model may determine a value (e.g., κ) of the gain factor 750 for a state factor (e.g., y(t-Δt)) to which the value of the timing factor 740 is applied. For example, κ may be determined based on the target exercise mode.

The target motion control model may determine a torque value (e.g., τ(t)) based on the target type of the target exercise mode. For example, when the target type is the first type (e.g., the step exercise type), the torque value may be determined based on Equation 7 below. For another example, when the target type is the second type (e.g., the non-step exercise type), the torque value may be determined based on Equation 8 below.

[Equation 7]

$$\tau_r(t) = -\kappa y(t - \Delta t)$$
$$\tau_l(t) = \kappa y(t - \Delta t)$$

[Equation 8]

$$\tau_r(t) = \tau_l(t) = \kappa y(t - \Delta t)$$

The target motion control model may control the wearable device based on the torque value. For example, the target motion control model may control the wearable device such that the wearable device outputs an assistance force or resistance force corresponding to the torque value to the user.

According to an example embodiment, when the wearable device includes only one joint, the torque value may be determined similarly to Equation 8.

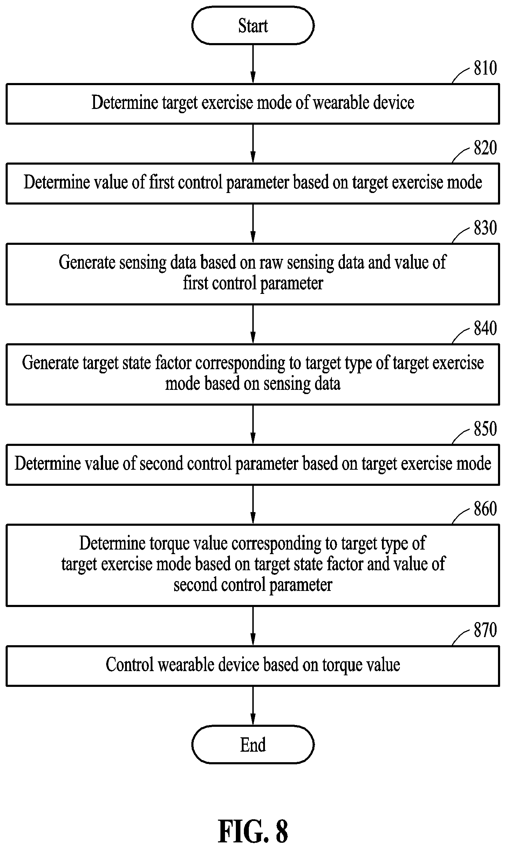

FIG. 8 is a flowchart illustrating an example method of controlling a wearable device based on an exercise mode according to an example embodiment.

Operations 810 to 870 described below may be performed by an electronic device (e.g., the electronic device 110 of FIG. 1, the wearable device 120 of FIG. 1, the electronic device 201 of FIG. 2, or the wearable device 300 of FIGS. 3A, 3B, and 3C).

In operation 810, a processor of the electronic device may determine a target exercise mode of a wearable device. For example, the target exercise mode may be an exercise mode that is being performed or executed currently by the wearable device in the course of an exercise program including one or more exercise modes. The exercise program may be a set of a plurality of exercise modes that are to be performed sequentially by a user. For example, when a previous exercise mode in a previous order set before an order of the target exercise mode among the exercise modes for which respective execution orders are set is performed or completed, the electronic device may determine the target exercise mode in a next order.

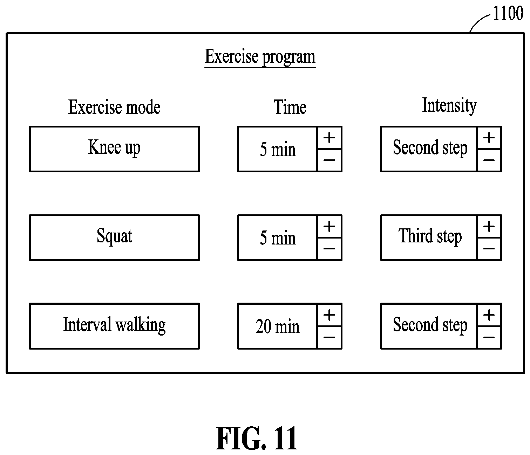

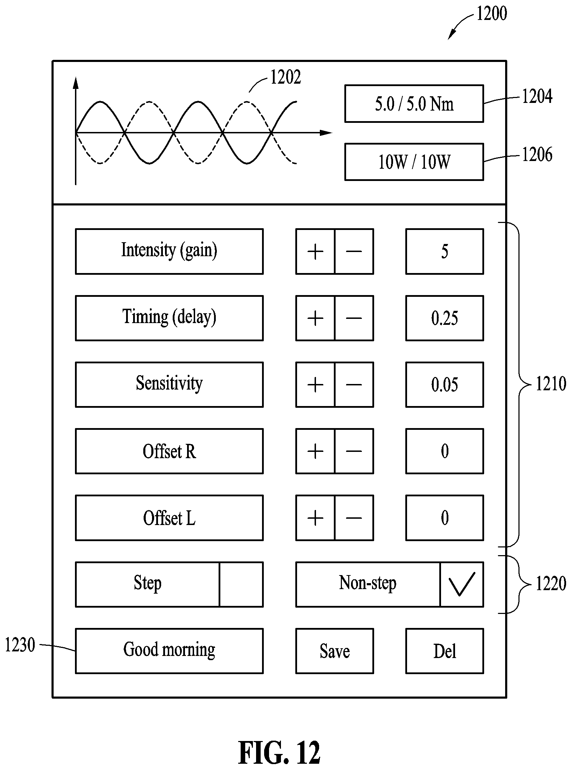

According to an example embodiment, the target exercise mode may be an exercise mode personalized in advance for a user of the wearable device. For example, the target exercise mode may be personalized through adjustment of at least one of a value of a first control parameter or a value of a second control parameter for the target exercise mode. A method of personalizing an exercise mode will be described in detail below with reference to FIGS. 11 and 12.

According to an example embodiment, the target exercise mode may be an exercise mode of a step exercise type. For example, the exercise mode of the step exercise type may include a walking exercise mode including, for example, power walking, interval walking, water walking, resisted walking, balance training walking, and assisted walking. For example, the exercise mode of the step exercise type may include a running exercise mode. For example, the exercise mode of the step exercise type may include an exercise mode in which a left leg and a right leg of the user move symmetrically or alternately, for example, a fast feet mode, a lunge mode, a split jacks mode, a toe-tap triceps mode, a knee up mode, a march step with twist mode, or a mountain climber mode.

According to an example embodiment, the target exercise mode may be an exercise mode of a non-step exercise type. For example, the exercise mode of the non-step exercise type may include an exercise mode in which a left leg and a right leg of the user move in the same direction, for example, a squat mode, a narrow squat mode, a half squat mode, a deadlift mode, a single leg deadlift mode, a kick back mode, a bird dog mode, or a good morning mode.

According to an example embodiment, when the electronic device is the wearable device (e.g., the wearable device 120 of FIG. 1 and/or the wearable device 300 of FIG. 3), the wearable device may determine the target exercise mode by receiving information on the target exercise mode of the wearable device from an external electronic device (e.g., the electronic device 110 of FIG. 1 and/or the electronic device 201 of FIG. 2) through the communication module.

According to an example embodiment, the electronic device may receive the information on the target exercise mode from the user through a UI and determine the target exercise mode based on the received information. For example, the electronic device may receive the information on the target exercise mode through a user input over a touch display. In this example, the user may select the target exercise mode that is currently being performed or is to be performed soon from among a plurality of exercise modes output on the touch display and may thereby transmit the information on the target exercise mode to the electronic device. For example, the electronic device may receive the information on the target exercise mode by receiving a user voice through a microphone. In this example, the electronic device may determine the target exercise mode corresponding to the voice. The touch display and/or the microphone may be a module included in the electronic device or a module included in an additional device (e.g., the additional device 130 shown in Fig. 1) wirelessly connected to the electronic device.

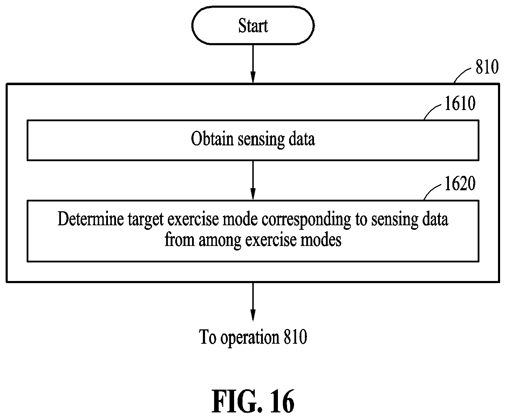

According to an example embodiment, the electronic device may actively determine the target exercise mode of the wearable device based on sensing data (e.g., raw sensing data) obtained through the wearable device while the user is doing an exercise with the wearable device worn on the user. A method by which the electronic device actively determines the target exercise mode will be described in detail below with reference to FIG. 16.

In operation 820, the processor of the electronic device may determine a value of a first control parameter for processing raw sensing data obtained from the wearable device, based on the target exercise mode.

According to an example embodiment, the first control parameter may include at least one of a parameter for adjusting the sensitivity of the raw sensing data or a parameter indicating an offset angle between joint angles of the raw sensing data. The raw sensing data may be sensing data that is unprocessed sensing data obtained through a corresponding sensor. For example, the raw sensing data may include a first raw angle (e.g., $q\_r\_raw(t)$) of a first joint (e.g., a right hip joint) and a second raw angle (e.g., $q\_l\_raw(t)$) of a second joint (e.g., $q\_r\_raw(t)$), as described above with reference to FIG. 7. For example, the first control parameter may include at least one of a sensitivity factor or an offset factor. For example, the value of the first control parameter may be a value personalized for the user.

According to an example embodiment, the value of the first control parameter may be determined based on a motion speed in the target exercise mode. For example, when the target exercise mode is an exercise mode for a fast motion such as a fast feet mode, a great value of the sensitivity of the raw sensing data may be determined as the first control parameter. For example, when the target exercise mode is an exercise mode for a slow motion such as a good morning mode, a small value of the sensitivity of the raw sensing data may be determined as the first control parameter.

According to an example embodiment, the value of the first control parameter may be determined based on a motion characteristic in the target exercise mode. For example, when the target exercise mode is a step exercise mode and requires a balance between a left leg and a right leg, a value of the offset angle may be determined as the first control parameter.

According to an example embodiment, when the determined target exercise mode is different from a previous target exercise mode, the processor of the electronic device may determine the value of the first control parameter such that a difference from a previous value of the first control parameter is within a preset range. For example, the value of the first control parameter may change stepwise or gradually during a preset time (e.g., an exercise mode transition time). As the value of the first control parameter changes gradually, a potential situation in which a value or direction of a torque to be output to the user changes drastically may be prevented or reduced.

According to an example embodiment, the preset time for an exercise mode transition may vary based on at least one of the previous exercise mode or the determined target exercise mode. For example, the transition time in a case in which the previous exercise mode and the target exercise mode are of the same exercise type may be longer than that in a case in which the previous exercise mode and the target exercise mode are of different exercise types. For example, the transition time in the case in which the previous exercise mode and the target exercise mode are of the same exercise type may be shorter than that in the case in which the previous exercise mode and the target exercise mode are of different exercise types.

In operation 830, the processor of the electronic device may generate sensing data based on the raw sensing data and the value of the first control parameter. For example, the raw sensing data may include the first raw angle (e.g., $q\_r\_raw(t)$) and the second raw angle (e.g., $q\_l\_raw(t)$). For example, the raw sensing data may include a first raw joint angle of a first leg and a second raw joint angle of a second leg that are generated by a sensor of the wearable device. For example, the sensing data may be a first angle (e.g., $q\_r(t)$) and a second angle (e.g., $q\_l(t)$), as described above with reference to FIG. 7. For example, the sensing data may be a first angle and a second angle to which an offset angle described above with reference to FIG. 7 is applied.

In operation 840, the processor of the electronic device may generate a target state factor corresponding to the target type of the target exercise mode based on the sensing data. The target state factor, which may be a state factor corresponding to the target type of the target exercise mode from between a state factor of a first type defined by Equation 5 or a state factor of a second type defined by Equation 6, may be a state factor that is currently used to control the wearable device.

For example, when the target type of the target exercise mode is the first type (e.g., the step exercise type), the target state factor may be generated based on Equation 5 described above with reference to FIG. 7. For another example, when the target type of the target exercise mode is the second type (e.g., the non-step exercise type), the target state factor may be generated based on Equation 6 described above with reference to FIG. 7.

In operation 850, the processor of the electronic device may determine a value of a second control parameter for at least one of an output timing or a magnitude of a torque to be output by the wearable device, based on the target exercise mode. The magnitude of the torque may include both an intensity and direction of the torque. For example, when the magnitude of the torque is "-1," the numeral "1" may denote the intensity of the torque and the sign "-" may denote the direction of the torque.

According to an example embodiment, the second control parameter may include at least one of a parameter for controlling the output timing of the torque output by the wearable device or a parameter for controlling the magnitude of the torque. For example, the second control parameter may include at least one of a timing factor or a gain factor. The gain factor may include the intensity and direction of the torque. For example, the value of the second control parameter may be a value personalized for the user.

According to an example embodiment, when the determined target exercise mode is different from the previous exercise mode, the processor of the electronic device may determine the value of the second control parameter such that a difference from a previous value of the second control parameter is within a preset range. For example, the value of the second control parameter may change stepwise or gradually during a preset time (e.g., an exercise mode transition time). As the value of the second control parameter changes gradually, a potential situation in which a value or direction of a torque to be output to the user changes drastically may be prevented or reduced.

According to an example embodiment, the preset time for an exercise mode transition may vary based on at least one of the previous exercise mode or the determined target exercise mode. For example, the transition time in a case in which the previous exercise mode and the target exercise mode are of the same exercise type may be longer than that in a case in which the previous exercise mode and the target exercise mode are of different exercise types. For example, the transition time in the case in which the previous exercise mode and the target exercise mode are of the same exercise type may be shorter than that in the case in which the previous exercise mode and the target exercise mode are of different exercise types.

In operation 860, the processor of the electronic device may determine a torque value corresponding to the target type of the target exercise mode based on the target state factor and the value of the second control parameter.

For example, when the target type is the first type (e.g., the step exercise type), the torque value may be determined based on Equation 7 described above with reference to FIG. 7. For another example, when the target type is the second type (e.g., the non-step exercise type), the torque value may be determined based on Equation 8 described above with reference to FIG. 7.

In operation 870, the electronic device may control the wearable device based on the determined torque value.

According to an example embodiment, when the electronic device is a user terminal, the electronic device may control the wearable device by transmitting information on the torque value to the wearable device. According to an example embodiment, when the electronic device is a wearable device, the electronic device may control the wearable device based on the determined torque value.

As the wearable device controls a motor disposed on a joint (e.g., a hip joint) of the wearable device based on the torque value, the wearable device may provide the user with a force corresponding to the torque value. The force may be, for example, an assistance force or a resistance force.

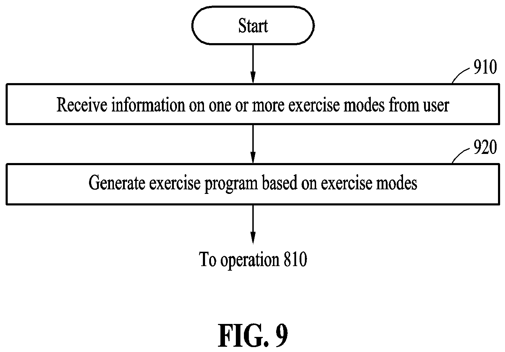

FIG. 9 is a flowchart illustrating an example method of generating an exercise program based on exercise modes according to an example embodiment.

According to an example embodiment, operations 910 and 920 described below may be performed by an electronic device (e.g., the electronic device 110 of FIG. 1, the wearable device 120 of FIG. 1, the electronic device 201 of FIG. 2, or the wearable device 300 of FIGS. 3A, 3B, and 3C). Operations 910 and 920 may be performed before operation 810 described above with reference to FIG. 8 is performed.

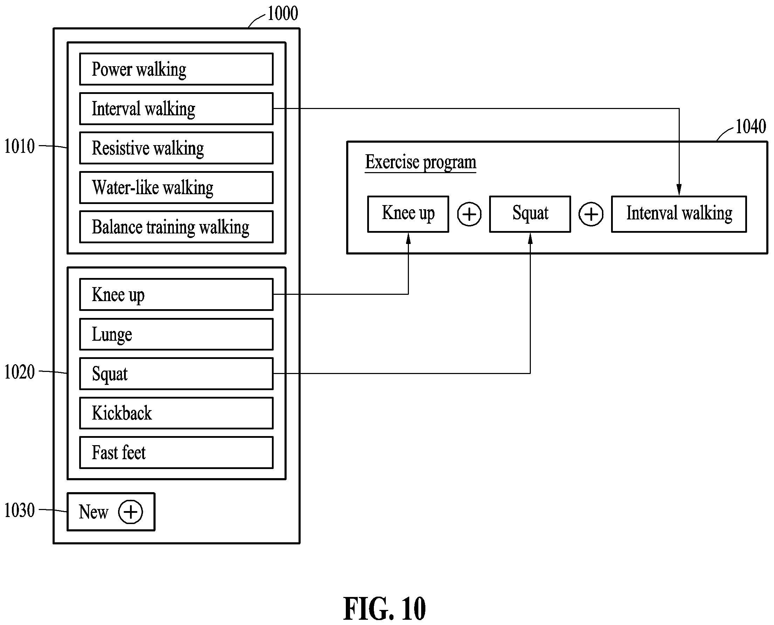

In operation 910, the electronic device may receive information on one or more exercise modes from the user. For example, the information on the exercise modes may include information on an execution time of an exercise mode and information on an execution level of the exercise mode. A method of receiving information on one or more exercise modes from a user will be described in greater detail below with reference to FIG. 10.

In operation 920, the electronic device may generate an exercise program based on the exercise modes.

According to an example embodiment, the electronic device may provide an exercise to the user by controlling the wearable device through the exercise program. For example, the wearable device may be controlled based on a motion control model for each of the exercise modes included in the exercise program.

FIG. 10 is a diagram illustrating an example method of generating an exercise program according to an example embodiment.

According to an example embodiment, an electronic device (e.g., the electronic device 110 of FIG. 1, the wearable device 120 of FIG. 1, the electronic device 201 of FIG. 2, or the wearable device 300 of FIGS. 3A, 3B, and 3C) may provide a user with a list 1000 on which a plurality of exercise modes is listed. For example, the list 1000 may include a first sub-list 1010 and a second sub-list 1020 that are divided by type. For example, the first sub-list 1010 may include one or more exercise modes of a step exercise type, and the second sub-list 1020 may include one or more exercise modes of a non-step exercise type.

According to an example embodiment, the list 1000 may further include a UI 1030 through which the user is able to generate a new exercise mode. For example, the user may generate the new exercise mode using the UI 1030. A method of generating a new exercise mode will be described in detail below with reference to FIG. 12.

According to an example embodiment, the user may generate an exercise program 1040 by selecting at least some of the exercise modes from the list 1000. For example, the user may generate the exercise program 1040 by selecting a knee up mode, a squat mode, and an interval walking mode from among the exercise modes on the list 1000.

According to an example embodiment, each of the exercise modes on the list 1000 may be an exercise mode personalized in advance. For example, values of control parameters of a motion control model for an exercise mode may be predetermined for the user. The values of the control parameters for the exercise mode may be determined by the user. The values of the control parameters for the exercise mode may be determined by the electronic device such that the values are suitable for the user.

FIG. 11 is a diagram illustrating an example of adjusting an execution time and level of each exercise mode of an exercise program according to an example embodiment.

According to an example embodiment, an electronic device (e.g., the electronic device 110 of FIG. 1, the wearable device 120 of FIG. 1, the electronic device 201 of FIG. 2, or the wearable device 300 of FIGS. 3A, 3B, and 3C) may receive, from a user, information on exercise modes included in an exercise program 1100. For example, the information may include information on an execution time of the exercise modes and information on an execution level of the exercise modes.

According to an example embodiment, a target level among a plurality of execution levels may be set for an exercise mode. For example, values of control parameters may be differently preset for each of the execution levels.

For example, for a knee up mode which is a first exercise mode, an execution time may be set to 5 minutes and an execution level may be set to 2 steps. For a squat mode which is a second exercise mode, an execution time may be set to 5 minutes and an execution level may be set to 3 steps. For an interval walking mode which is a third exercise mode, an execution time may be set to 20 minutes and an execution level may be set to 2 steps.

FIG. 12 is a diagram illustrating an example method of adjusting values of control parameters of an exercise mode according to an example embodiment.

According to an example embodiment, an electronic device (e.g., the electronic device 110 of FIG. 1, the wearable device 120 of FIG. 1, the electronic device 201 of FIG. 2, or the wearable device 300 of FIGS. 3A, 3B, and 3C) may provide a user with a UI 1200 through which the user is able to adjust values of control parameters of an exercise mode. For example, the UI 1200 may be an authoring tool for generating an exercise mode.

According to an example embodiment, the user may adjust the values of the control parameters by changing values of the control parameters set for an existing exercise mode.

According to an example embodiment, the user may set values of the control parameters of a new exercise mode while generating the new exercise mode. For example, the user may select the UI 1030 described above with reference to FIG. 10 to generate the new exercise mode, and when the UI 1030 is selected, the UI 1200 may be provided to the user.

The new exercise mode may be generated by changing the values of the control parameters using the existing exercise mode. For example, the user may generate a fast feet mode by adjusting values of a sensitivity factor and a timing factor of a basic walking exercise mode. For example, to generate a good morning mode or a deadlift mode, the user may adjust values of the control parameters of a squat mode with similar waist motions.

For example, the UI 1200 may include a graph 1202 showing a trajectory of joint angles shown in a corresponding exercise mode and a trajectory of an output torque.

For example, the UI 1200 may output a torque value 1204 that is output to a left joint and a right joint. The torque value 1204 may be an instantaneous maximum or large torque value, or a root mean square (RMS) torque value.

For example, the UI 1200 may output a value 1206 of power generated by a force provided to the user.

For example, the UI 1200 may include a UI 1210 for adjusting the values of the control parameters.

For example, the UI 1200 may include a UI 1220 for setting a type of a corresponding exercise mode.

For example, the UI 1200 may include a UI 1230 for setting a name of a corresponding exercise mode.

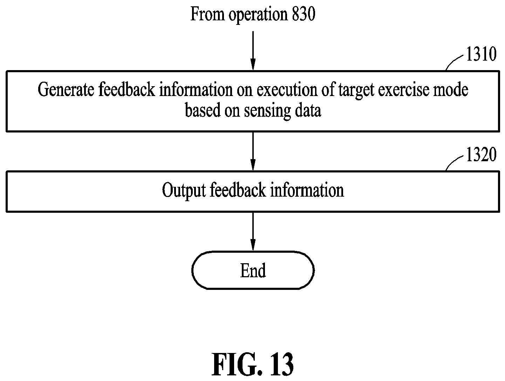

FIG. 13 is a flowchart illustrating an example method of outputting feedback information generated based on sensing data according to an example embodiment.

According to an example embodiment, operations 1310 and 1320 described below may be performed by an electronic device (e.g., the electronic device 110 of FIG. 1, the wearable device 120 of FIG. 1, the electronic device 201 of FIG. 2, or the wearable device 300 of FIGS. 3A, 3B, and 3C). Operation 1310 may be performed after operation 830 described above with reference to FIG. 8 is performed.

In operation 1310, the electronic device may generate feedback information on execution of a target exercise mode based on sensing data.

According to an example embodiment, the electronic device may determine in real time a present pose that is currently taken by a user doing an exercise based on the sensing data and may compare the present pose of the user and a target pose preset for the target exercise mode. The electronic device may generate, as the feedback information, a difference between the present pose and the target pose. For example, the present pose of the user may be determined based on an angle of a hip joint, a range of motion of the hip joint, or IMU information measured by an IMU (e.g., the IMU 330 of FIGS. 3A, 3B, 3C, and 3D).

According to an example embodiment, when a wearable device operates with values of control parameters for the target exercise mode, the electronic device may calculate a value of power provided to the user and generate the calculated value of power as the feedback information. For example, in an assistance mode, a positive value of power may be calculated. For another example, in a resistance mode, a negative value of power may be calculated.

According to an example embodiment, the electronic device may calculate a first RMS torque based on a first torque. For example, the electronic device may calculate the first RMS torque with respect to output torque values. For example, the first RMS torque may be calculated based on Equation 9 below.

[Equation 9]

$$\tau_{RMS} = \sqrt{\frac{1}{n}\sum_{i=1}^{n}\tau_i{}^2}$$

In Equation 9, n denotes a total number of data obtained by a one-time motion of the user, $\tau_i$ denotes an ith torque, and $\tau_{RMS}$ denotes an RMS torque for the one-time motion.

The electronic device may calculate a first average value of power based on a first value of power. The first value of power may be a value of power generated as the first torque is output. The first value of power may be calculated as a product between the first torque $\tau_i$ and a first angular velocity $\dot{q}_i$. The first angular velocity may be obtained based on first sensing data of a first joint.

The electronic device may calculate the first average value of power based on output torques and angular velocities obtained at a time when the torques are output. For example, the first average value of power may be calculated based on the first value of power and a second value of power at a second time that is later than a first time. The first average value of power may be calculated based on Equation 10 below.

[Equation 10]

$$MP = {1}/{n}\sum_{i=1}^{n}\tau_i\dot{q}_i$$

In Equation 10, n denotes a total number of data obtained by a one-time motion of the user, $\tau_i$ denotes an ith torque (e.g., ith time or sample), $\dot{q}_i$ denotes an ith angular velocity, and MP denotes an average value of power for the one-time motion.

According to an example embodiment, the electronic device may calculate, as the feedback information, a value of output power in comparison to an input torque by calculating $MP/\tau_{RMS}$. For example, $MP/\tau_{RMS}$ denotes agility of a motion. In this example, a unit of the calculated value of the agility of the motion is rad/sec.

In operation 1320, the electronic device may output the feedback information. For example, the electronic device may visually output the feedback information over a display. For example, the electronic device may audibly output the feedback information through a speaker. The feedback information may output through an additional device (e.g., the additional device 130 of FIG. 1) connected to the electronic device, in addition to the display or a speaker of the electronic device.

According to an example embodiment, the user wearing the wearable device may check the accuracy of the exercise they are doing, based on the feedback information. For example, the user may modify or change an exercise pose taken by the user based on the feedback information.

According to an example embodiment, a trainer who instructs the user wearing the wearable device on how to do the exercise may check the feedback information through the electronic device. The trainer may determine the suitability of the values of the control parameters for the target exercise mode performed by the user, based on the value of power as the feedback information. For example, when it is determined that the values of the control parameters for the target exercise mode are not suitable, the trainer may adjust the values of the control parameters.

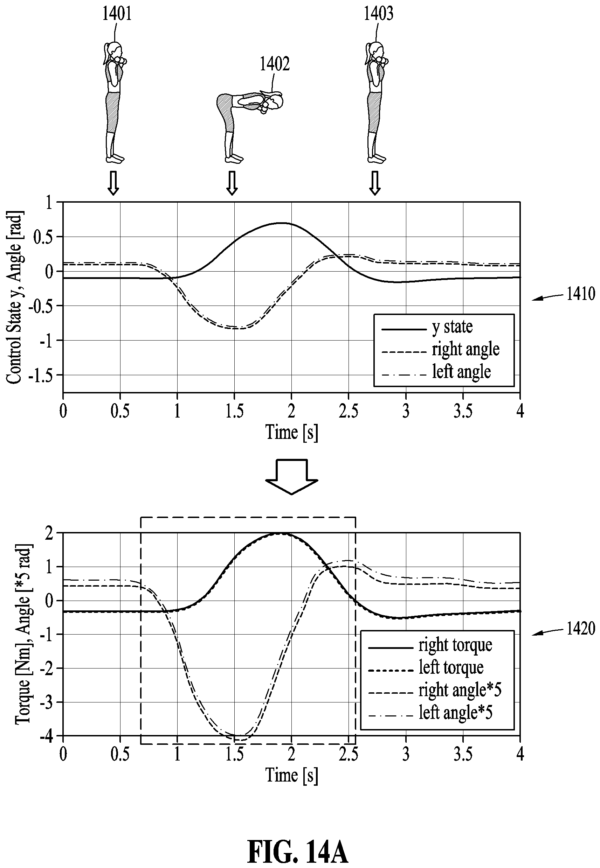

FIG. 14A is a diagram illustrating an example trajectory of joint angles and an example trajectory of a state factor with respect to the joint angles in a target exercise mode, and an example trajectory of a torque value determined based on the state factor and an assistance mode according to an example embodiment.

According to an example embodiment, a graph 1410 shows a traj ectory of j oint angles corresponding to a change in motions 1401, 1402, and 1403 performed by a user and a trajectory of a state factor determined based on the joint angles, when a good morning mode is performed as an exercise mode. For example, the good morning mode may be of a non-step exercise type, and thus the state factor for the good morning mode may be generated based on Equation 6 described above with reference to FIG. 7.

According to an example embodiment, in a case in which the good morning mode operates as an assistance mode, when the user performs motions while changing from the motion 1402 of bending their waist to the motion 1403 of stretching the waist, a torque that assists the user in stretching the waist may be provided as an assistance force to the user. In addition, a graph 1420 shows a trajectory of a torque value, in which a positive value of torque may indicate the assistance force that assists the user in stretching the waist.

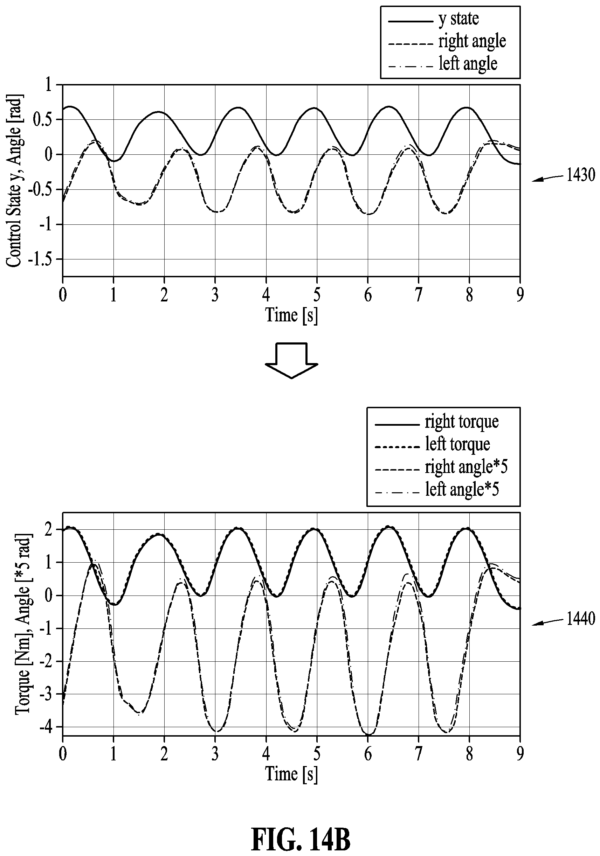

FIG. 14B is a diagram illustrating an example trajectory of joint angles measured when a target exercise of FIG. 14A is repeatedly performed and an example trajectory of a state factor with respect to the joint angles, and an example trajectory of a torque value determined based on the state factor and an assistance mode according to an example embodiment.

According to an example embodiment, a graph 1430 shows a trajectory of joint angles corresponding to a change in motions performed by a user and a trajectory of a state factor determined based on the joint angles, when the user repeatedly performs a good morning exercise.

According to an example embodiment, a graph 1440 shows a trajectory of a torque value determined based on the state factor and an assistance mode, when a good morning mode operates as an assistance mode.

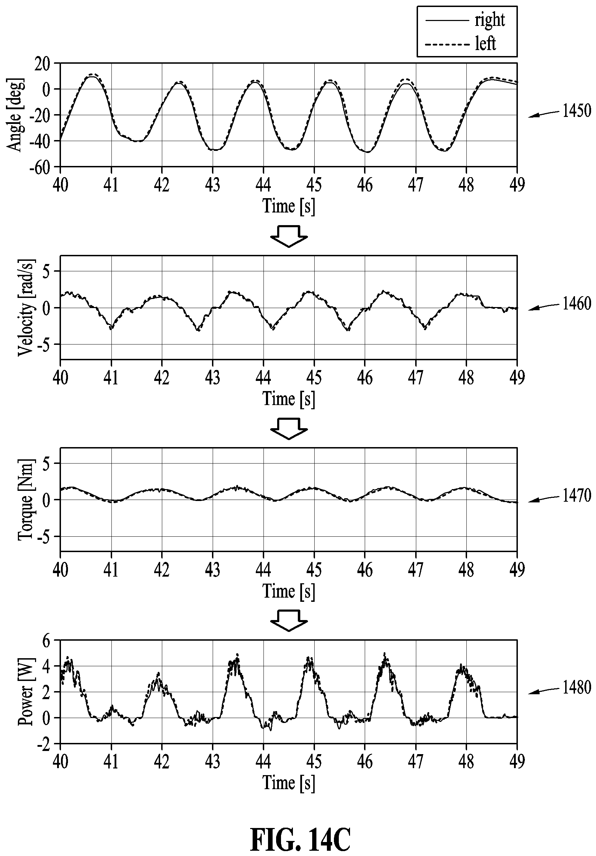

FIG. 14C is a diagram illustrating an example trajectory of a torque value output by a wearable device and an example trajectory of power, when a target exercise of FIG. 14A is repeatedly performed according to an example embodiment.

According to an example embodiment, in a case in which a user repeatedly performs a good morning exercise, a graph 1450 shows a trajectory of joint angles corresponding to a change in motions performed by the user, a graph 1460 shows a trajectory of a joint angular velocity, a graph 1470 shows a trajectory of a torque value, and a graph 1480 shows a trajectory of a power value. In the graph 1480, a positive value of power may indicate that an assistance force is provided to the user, and a negative value of power may indicate that a resistance force is provided to the user. In a good morning exercise mode as an assistance mode, it may be verified that the assistance force is provided to the user in most sections.

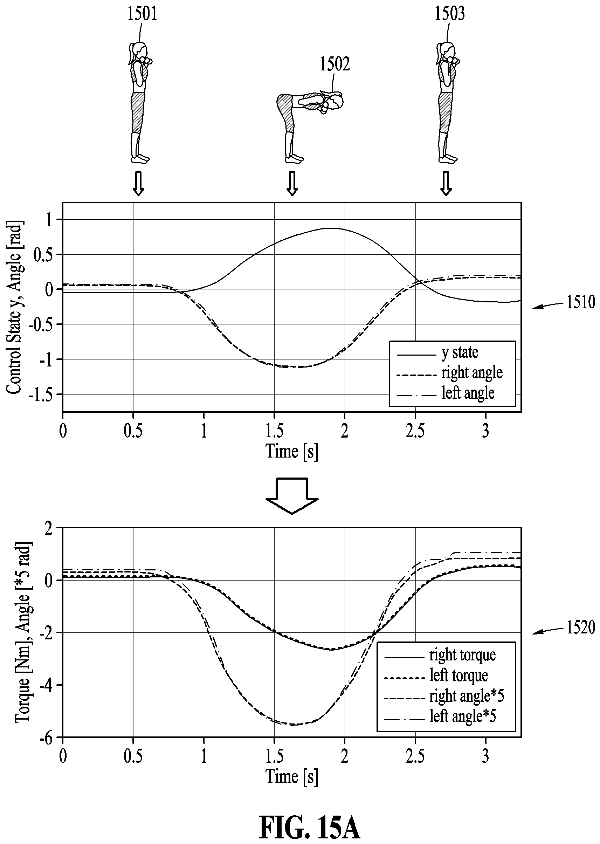

FIG. 15A is a diagram illustrating an example trajectory of joint angles in a target exercise mode and an example trajectory of a state factor with respect to the joint angles, and an example trajectory of a torque value determined based on the state factor and a resistance mode according to an example embodiment.

According to an example embodiment, in a case in which a good morning mode is performed as an exercise mode, a graph 1510 shows a trajectory of joint angles corresponding to a change in motions 1501, 1502, and 1503 performed by a user and a trajectory of a state factor determined based on the joint angles.

According to an example embodiment, in a case in which the good morning mode operates as a resistance mode, when the user performs motions while changing from the motion 1502 of bending their waist to the motion 1503 of stretching the waist, a torque that hinders the user from stretching the waist may be provided as a resistance force to the user. In addition, a graph 1520 shows a trajectory of a torque value, in which a negative value of torque may indicate the resistance force that hinders the user from stretching the waist.

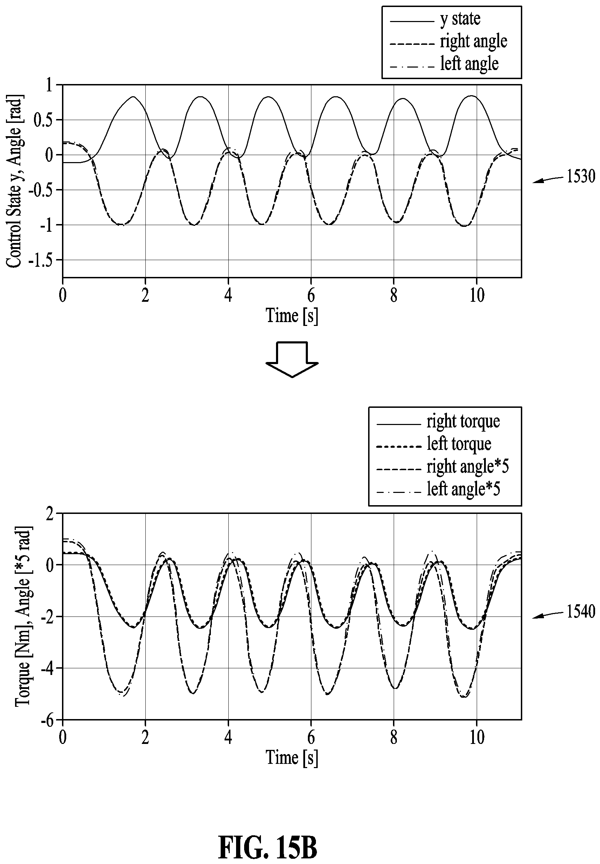

FIG. 15B is a diagram illustrating an example trajectory of joint angles measured when a target exercise of FIG. 15A is repeatedly performed and an example trajectory of a state factor with respect to the joint angles, and an example trajectory of a torque value determined based on the state factor and a resistance mode according to an example embodiment.

According to an example embodiment, a graph 1530 shows a trajectory of joint angles corresponding to a change in motions performed by a user and a trajectory of a state factor determined based on the joint angles, when the user repeatedly performs a good morning exercise.

According to an example embodiment, a graph 1540 shows a trajectory of a torque value determined based on the state factor and a resistance mode, when a good morning mode operates as a resistance mode.

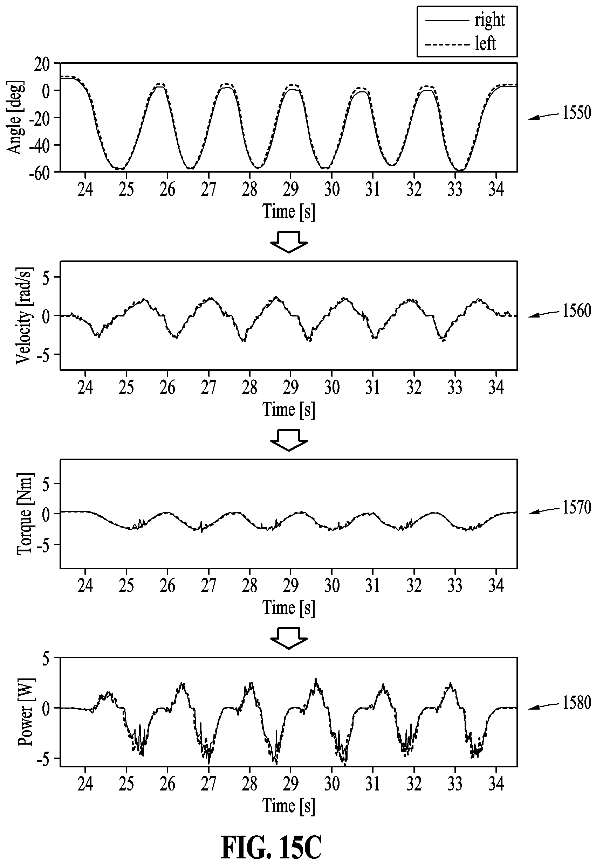

FIG. 15C is a diagram illustrating an example trajectory of a torque value output by a wearable device and an example trajectory of power, when a target exercise of FIG. 15A is repeatedly performed according to an example embodiment.

According to an example embodiment, in a case in which a user repeatedly performs a good morning exercise, a graph 1550 shows a trajectory of joint angles corresponding to a change in motions performed by the user, a graph 1560 shows a trajectory of a joint angular velocity, a graph 1570 shows a trajectory of a torque value, and a graph 1580 shows a trajectory of a power value. In the graph 1580, a negative value of power may indicate that a resistance force is provided to the user, and a positive value of power may indicate that an assistance force is provided to the user. In a good morning exercise mode as a resistance mode, it may be verified that the resistance force is provided to the user in most sections.

FIG. 16 is a flowchart illustrating an example method in which an electronic device actively determines a target exercise mode according to an example embodiment.

According to an example embodiment, operation 810 described above with reference to FIG. 8 may include operations 1610 and 1620 described below. Operations 1610 and 1620 may be performed by an electronic device (e.g., the electronic device 110 of FIG. 1, the wearable device 120 of FIG. 1, the electronic device 201 of FIG. 2, and/or the wearable device 300 of FIGs. 3A-3C).

In operation 1610, the electronic device may obtain sensing data (e.g., raw sensing data) through a sensor of a wearable device while a user is doing an exercise with the wearable device worn on the user. The sensing data may include, for example, a joint angle, a joint angular velocity, and/or IMU information. For example, when the sensing data is the joint angle (or the joint angular velocity and/or the IMU information), the sensing data may further include a trajectory (e.g., a pattern) of a change in the joint angle (and/or the joint angular velocity, and/or the IMU information) over time.

In operation 1620, the electronic device may determine a target exercise mode corresponding to the sensing data from among a plurality of exercise modes. For example, the electronic device may determine the target exercise mode corresponding to the sensing data based on a preset rule. For example, the electronic device may determine the target exercise mode based on an exercise mode classification model trained in advance to classify the exercise modes based on the sensing data. The exercise mode classification model may be a neural network-based classification model. The exercise mode classification model may be trained in advance to identify an exercise mode corresponding to input sensing data, based on a plurality of pieces of sensing data respectively corresponding to a plurality of exercise modes.

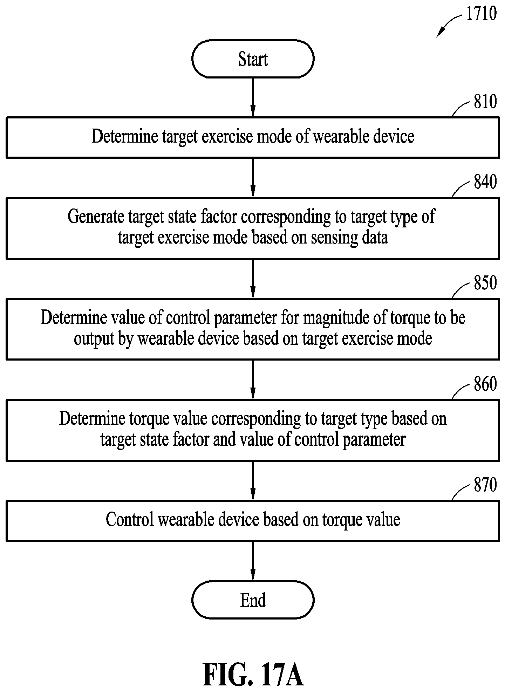

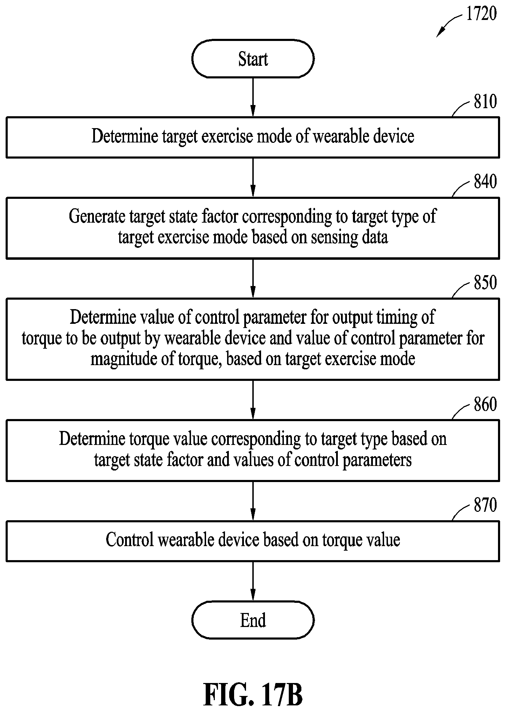

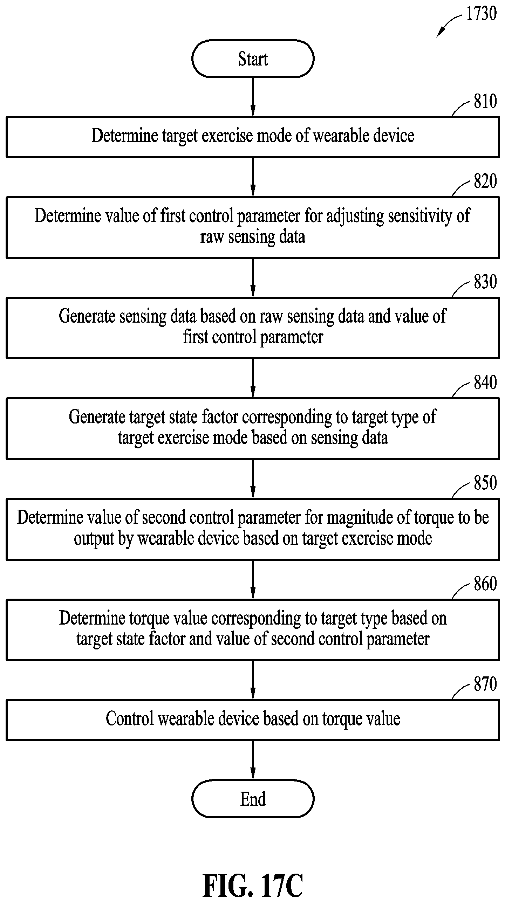

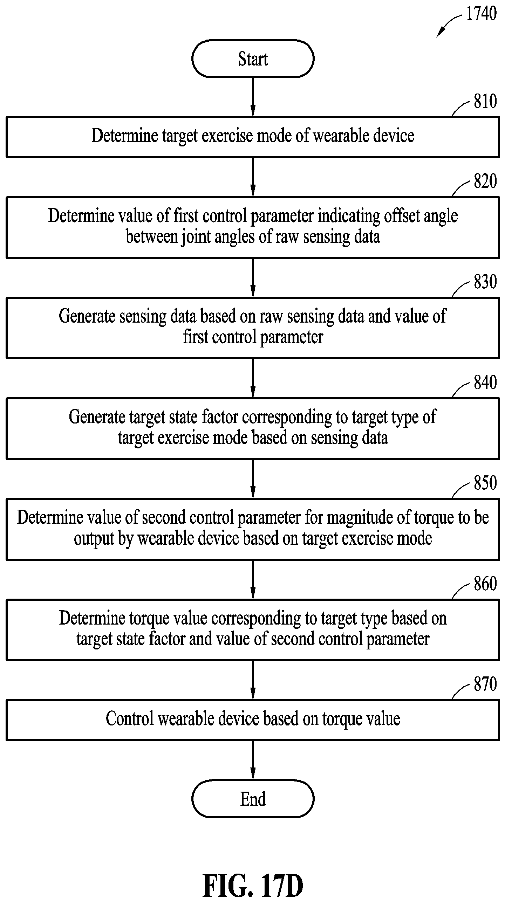

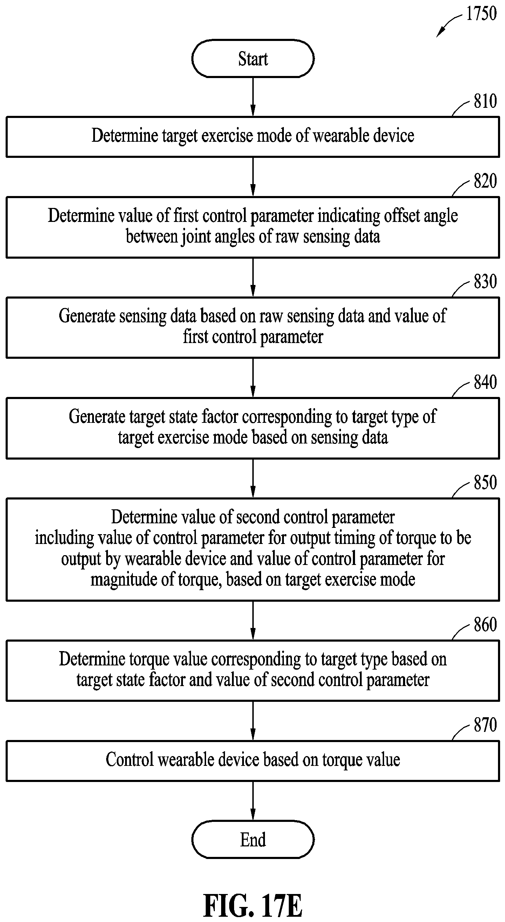

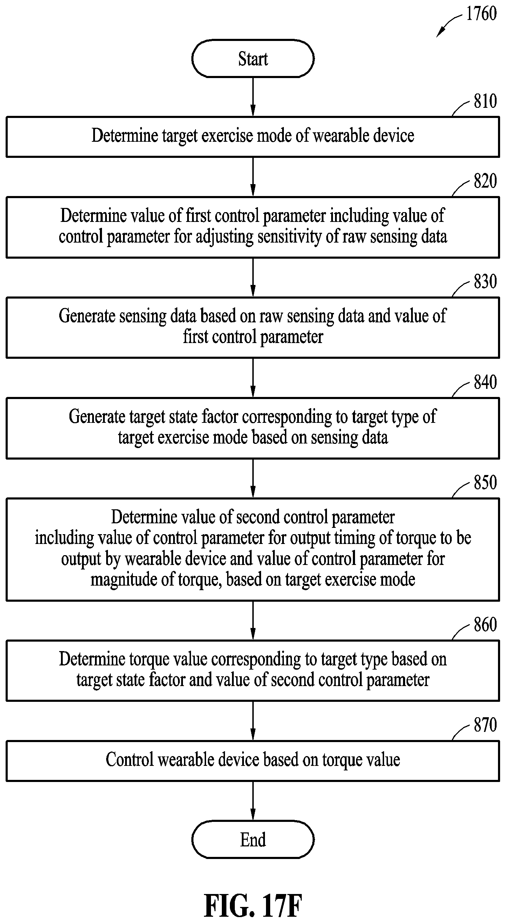

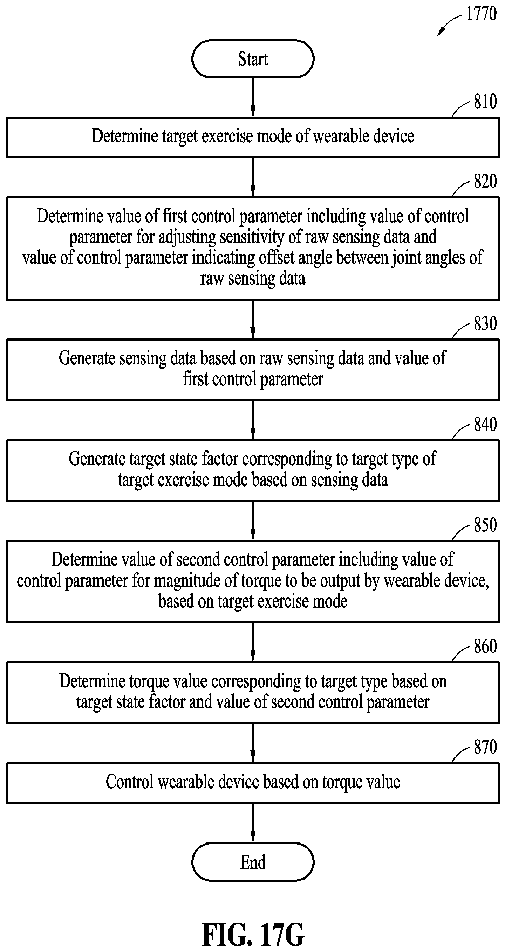

FIGS. 17A, 17B, 17C, 17D, 17E, 17F, and 17G are each a flowchart illustrating an example method of controlling a wearable device based on an exercise mode according to an example embodiment.

At least some of operations 810 to 870 sequentially described above with reference to FIG. 8 may be omitted from an example of implementation according to an example embodiment.

In a method 1710 illustrated with reference to FIG. 17A, at least one processor of an electronic device may perform operation 810 of determining a target exercise mode of a wearable device, operation 840 of generating a target state factor corresponding to a target type of the target exercise mode based on sensing data (or raw sensing data) obtained from the wearable device, operation 850 of determining a value of a second control parameter for a magnitude of a torque to be output by the wearable device based on the target exercise mode, operation 860 of determining a torque value corresponding to the target type of the target exercise mode based on the target state factor and the value of the second control parameter, and operation 870 of controlling the wearable device based on the torque value.

In a method 1720 illustrated with reference to FIG. 17B, the at least one processor of the electronic device may perform operation 810 of determining a target exercise mode of a wearable device, operation 840 of generating a target state factor corresponding to a target type of the target exercise mode based on sensing data (or raw sensing data) obtained from the wearable device, operation 850 of determining a value of a second control parameter including a value of a control parameter for an output timing of a torque to be output by the wearable device and a value of a control parameter for a magnitude of the torque based on the target exercise mode, operation 860 of determining a torque value corresponding to the target type of the target exercise mode based on the target state factor and the value of the second control parameter, and operation 870 of controlling the wearable device based on the torque value.

In a method 1730 illustrated with reference to FIG. 17C, the at least one processor of the electronic device may perform operation 810 of determining a target exercise mode of a wearable device, operation 820 of determining a value of a first control parameter including a control parameter for adjusting a sensitivity of raw sensing data obtained from the wearable device based on the target exercise mode, operation 830 of generating sensing data based on the raw sensing data and the value of the first control parameter, operation 840 of generating a target state factor corresponding to a target type of the target exercise mode based on the sensing data, operation 850 of determining a value of a second control parameter for a magnitude of a torque to be output by the wearable device based on the target exercise mode, operation 860 of determining a torque value corresponding to the target type of the target exercise mode based on the target state factor and the value of the second control parameter, and operation 870 of controlling the wearable device based on the torque value.

In a method 1740 illustrated with reference to FIG. 17D, the at least one processor of the electronic device may perform operation 810 of determining a target exercise mode of a wearable device, operation 820 of determining a value of a first control parameter including a control parameter indicating an offset angle between joint angles of raw sensing data obtained from the wearable device based on the target exercise mode, operation 830 of generating sensing data based on the raw sensing data and the value of the first control parameter, operation 840 of generating a target state factor corresponding to a target type of the target exercise mode based on the sensing data, operation 850 of determining a value of a second control parameter for a magnitude of a torque to be output by the wearable device based on the target exercise mode, operation 860 of determining a torque value corresponding to the target type of the target exercise mode based on the target state factor and the value of the second control parameter, and operation 870 of controlling the wearable device based on the torque value.

In a method 1750 illustrated with reference to FIG. 17E, the at least one processor of the electronic device may perform operation 810 of determining a target exercise mode of a wearable device, operation 820 of determining a value of a first control parameter including a control parameter indicating an offset angle between joint angles of raw sensing data obtained from the wearable device based on the target exercise mode, operation 830 of generating sensing data based on the raw sensing data and the value of the first control parameter, operation 840 of generating a target state factor corresponding to a target type of the target exercise mode based on the sensing data, operation 850 of determining a value of a second control parameter including a value of a control parameter for an output timing of a torque to be output by the wearable device and a value of a control parameter for a magnitude of the torque based on the target exercise mode, operation 860 of determining a torque value corresponding to the target type of the target exercise mode based on the target state factor and the value of the second control parameter, and operation 870 of controlling the wearable device based on the torque value.

In a method 1760 illustrated with reference to FIG. 17F, the at least one processor of the electronic device may perform operation 810 of determining a target exercise mode of a wearable device, operation 820 of determining a value of a first control parameter including a control parameter for adjusting a sensitivity of raw sensing data obtained from the wearable device based on the target exercise mode, operation 830 of generating sensing data based on the raw sensing data and the value of the first control parameter, operation 840 of generating a target state factor corresponding to a target type of the target exercise mode based on the sensing data, operation 850 of determining a value of a second control parameter including a value of a control parameter for an output timing of a torque to be output by the wearable device and a value of a control parameter for a magnitude of the torque based on the target exercise mode, operation 860 of determining a torque value corresponding to the target type of the target exercise mode based on the target state factor and the value of the second control parameter, and operation 870 of controlling the wearable device based on the torque value.

In a method 1770 illustrated with reference to FIG. 17G, the at least one processor of the electronic device may perform operation 810 of determining a target exercise mode of a wearable device, operation 820 of determining a value of a first control parameter including a control parameter for adjusting a sensitivity of raw sensing data obtained from the wearable device and a control parameter indicating an offset angle between joint angles of the raw sensing data based on the target exercise mode, operation 830 of generating sensing data based on the raw sensing data and the value of the first control parameter, operation 840 of generating a target state factor corresponding to a target type of the target exercise mode based on the sensing data, operation 850 of determining a value of a second control parameter including a value of a control parameter for a magnitude of a torque to be output by the wearable device based on the target exercise mode, operation 860 of determining a torque value corresponding to the target type of the target exercise mode based on the target state factor and the value of the second control parameter, and operation 870 of controlling the wearable device based on the torque value.

According to an example embodiment, an electronic device may include a communication module configured to exchange data with an external device, and at least one processor (including processing circuitry) configured to control the electronic device. The processor may determine a target exercise mode of a wearable device, determine a value of a first control parameter based on the target exercise mode, generate sensing data based on raw sensing data obtained from the wearable device and the value of the first control parameter, generate a target state factor corresponding to a target type of the target exercise mode based on the sensing data, determine a value of a second control parameter based on the target exercise mode, determine a torque value corresponding to the target type of the target exercise mode based on the target state factor and the value of the second control parameter, and control the wearable device based on the torque value.

According to an example embodiment, the target type of the target exercise mode may be any one of a step exercise type and a non-step exercise type.

According to an example embodiment, the determining of the target exercise mode of the wearable device may include determining the target exercise mode from among one or more exercise modes of an exercise program performed by the wearable device.

According to an example embodiment, the determining of the target exercise mode from among the exercise modes of the exercise program performed by the wearable device may include, when a previous exercise mode set in an order before an order of the target exercise mode among the exercise modes for which an execution order is set is performed, determining the target exercise mode in a next order.

According to an example embodiment, the first control parameter may include at least one of a parameter for adjusting sensitivity of the raw sensing data or a parameter indicating an offset angle between joint angles of the raw sensing data.

According to an example embodiment, the second control parameter may include at least one of a parameter for controlling an output timing of a torque output by the wearable device and a parameter for controlling a magnitude of the torque.

According to an example embodiment, the raw sensing data may include a first raw joint angle of a first leg and a second raw joint angle of a second leg that are generated by a sensor of the wearable device.

According to an example embodiment, the target exercise mode may be an exercise mode personalized for the user of the wearable device.

According to an example embodiment, the target exercise mode may be personalized by adjusting at least one of the value of the first control parameter or the value of the second control parameter for the target exercise mode.

According to an example embodiment, the processor may further receive information on one or more exercise modes including the target exercise mode from the user and generate an exercise program based on the exercise modes.

According to an example embodiment, the information on the exercise modes may include information on an execution time of the target exercise mode and information on an execution level of the target exercise mode.

According to an example embodiment, the processor may further generate feedback information on the execution of the target exercise mode based on the sensing data and output the feedback information.

According to an example embodiment, a force corresponding to the torque value may be output by the wearable device.

According to an example embodiment, a method of controlling a wearable device performed by an electronic device may include determining a target exercise mode of the wearable device, determining a value of a first control parameter based on the target exercise mode, generating sensing data based on raw sensing data obtained from the wearable device and the value of the first control parameter, generating a target state factor corresponding to a target type of the target exercise mode based on the sensing data, determining a value of a second control parameter based on the target exercise mode, determining a torque value corresponding to the target type of the target exercise mode based on the target state factor and the value of the second control parameter, and controlling the wearable device based on the torque value.

According to an example embodiment, the target type of the target exercise mode may be any one of a step exercise type and a non-step exercise type.

According to an example embodiment, the target exercise mode may be an exercise mode personalized for a user of the wearable device, and the target exercise mode may be personalized by adjusting at least one of the value of the first control parameter or the value of the second control parameter for the target exercise mode.

According to an example embodiment, the method of controlling the wearable device may further include receiving information on one or more exercise modes including the target exercise mode from the user and generating an exercise program based on the exercise modes.

According to an example embodiment, the information on the exercise modes may include information on an execution time of the target exercise mode and information on an execution level of the target exercise mode.

Each embodiment herein may be used in combination with any other embodiment(s) described herein.

According to an example embodiment, the method of controlling the wearable device may further include generating feedback information on the execution of the target exercise mode based on the sensing data and outputting the feedback information.

According to an example embodiment, an electronic device may include a communication module, including communication circuitry, configured to exchange data with an external device, and at least one processor configured to control the electronic device. The processor may receive information on one or more exercise modes from a user, generate an exercise program based on the exercise modes, determine a target exercise mode when the wearable device is controlled based on the exercise program, determine a value of a first control parameter based on the target exercise mode, generate sensing data based on raw sensing data of the wearable device and a value of the first control parameter, generate a target state factor corresponding to a target type of the target exercise mode based on the sensing data, determine a value of a second control parameter based on the target exercise mode, determine a torque value corresponding to the target type of the target exercise mode based on the target state factor and the value of the second control parameter, and control the wearable device based on the torque value. "Based on" as used herein covers based at least on.

The examples described herein may be implemented using hardware components, software components and/or combinations thereof. A processing device may be implemented using one or more general-purpose or special purpose computers, such as, for example, a processor, a controller and an arithmetic logic unit (ALU), a digital signal processor, a microcomputer, a field programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such as parallel processors. Each "processor" herein comprises processing circuitry.

Software may include a computer program, a piece of code, an instruction, or some combination thereof, to independently or collectively instruct or configure the processing device to operate as desired. Software and/or data may be embodied permanently or temporarily in any type of machine, component, physical or virtual equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described examples may be recorded in non-transitory computer-readable media including program instructions to implement various operations of the above-described examples. The media may also include, alone or in combination with the program instructions, data files, data structures, and the like. The program instructions recorded on the media may be those specially designed and constructed for the purposes of examples, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher-level code that may be executed by the computer using an interpreter.

The above-described hardware devices may be configured to act as one or more software modules in order to perform the operations of the above-described examples, or vice versa. The term "software module" as used herein may include various processing circuitry and/or executable program instructions. The same applies to "software modules."

While this disclosure includes specific examples, it will be apparent to one of ordinary skill in the art that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components or their equivalents.

Therefore, the scope of the disclosure is defined not by the detailed description, but by the claims and their equivalents, and all variations within the scope of the claims and their equivalents are to be construed as being included in the disclosure. While the disclosure has been illustrated and described with reference to various embodiments, it will be understood that the various embodiments are intended to be illustrative, not limiting. It will further be understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

**Claims**

1. An electronic device, comprising:

   a communication module, comprising communication circuitry, configured to exchange data with an external device; and
   at least one processor configured to control the electronic device,
   wherein the at least one processor is configured to:

determine a target exercise mode of a wearable device;
determine a value of a first control parameter for processing raw sensing data to be obtained from the wearable device, based on the target exercise mode;
generate sensing data based on the raw sensing data and the value of the first control parameter;
generate a target state factor corresponding to a target type of the target exercise mode based on the sensing data;
determine a value of a second control parameter for at least one of an output timing of a torque to be output by the wearable device or a magnitude of the torque, based on the target exercise mode;
determine a torque value corresponding to the target type of the target exercise mode based on the target state factor and the value of the second control parameter; and
control the wearable device based on the torque value.

2. The electronic device of claim 1, wherein the target type of the target exercise mode comprises one of a step exercise type and a non-step exercise type.

3. The electronic device of claim 1, wherein the at least one processor is configured to:
determine the target exercise mode from among one or more exercise modes of an exercise program performed by the wearable device.

4. The electronic device of claim 3, wherein the at least one processor is configured to:
when a previous exercise mode in an order set before an order of the target exercise mode among the exercise modes for which an execution order is set is performed, determine the target exercise mode in a next order.

5. The electronic device of claim 1, wherein the first control parameter comprises at least one of a parameter for adjusting a sensitivity of the raw sensing data, or a parameter indicating an offset angle between joint angles of the raw sensing data.

6. The electronic device of claim 1, wherein the second control parameter comprises at least one of a parameter for controlling the output timing of the torque output by the wearable device, or a parameter for controlling the magnitude of the torque.

7. The electronic device of claim 1, wherein the raw sensing data comprises a first raw joint angle of a first leg and a second raw joint angle of a second leg, generated by a sensor of the wearable device.

8. The electronic device of claim 1, wherein the target exercise mode comprises an exercise mode personalized for a user of the wearable device.

9. The electronic device of claim 8, wherein the target exercise mode is configured to be personalized via adjustment of at least one of the value of the first control parameter or the value of the second control parameter for the target exercise mode.

10. The electronic device of claim 1, wherein the processor is further configured to:

receive information regarding one or more exercise modes comprising the target exercise mode from a user; and
generate an exercise program based on the one or more exercise modes.

11. The electronic device of claim 10, wherein the information regarding the one or more exercise modes comprises information regarding an execution time of the target exercise mode and information regarding an execution level of the target exercise mode.

12. The electronic device of claim 1, wherein the processor is further configured to:

generate feedback information regarding execution of the target exercise mode based on the sensing data; and
control to output the feedback information.

13. The electronic device of claim 1, wherein a force corresponding to the torque value is to be output by the wearable device.

**14.** A method of controlling a wearable device performed by an electronic device in communication with the wearable device, the method comprising:

determining a target exercise mode of the wearable device;

determining a value of a first control parameter for processing raw sensing data obtained from the wearable device, based on the target exercise mode;

generating sensing data based on the raw sensing data and the value of the first control parameter;

generating a target state factor corresponding to a target type of the target exercise mode based on the sensing data;

determining a value of a second control parameter for at least one of an output timing of a torque to be output by the wearable device or a magnitude of the torque, based on the target exercise mode;

determining a torque value corresponding to the target type of the target exercise mode based on the target state factor and the value of the second control parameter; and

controlling the wearable device based on the torque value.

**15.** The method of claim 14, wherein the target type of the target exercise mode comprises one of a step exercise type and a non-step exercise mode.

**FIG. 1**

FIG. 2

EP 4 364 711 A1

**FIG. 3A**

**FIG. 3B**

300

| Memory 344 | Communication module 352 | Input interface 346 | Battery 350 |

| Sensor 321 | Processor 342 | Motor driver circuit 312 |

Motor 314

**FIG. 3C**

300-1

Battery
350

Memory
344

Input interface
346

Motor driver
circuit 312

Processor
342

Motor driver
circuit 312-1

Motor
314

Motor
314-1

Sensor
321

Sensor
321-1

## FIG. 3D

**FIG. 4**

**FIG. 5**

**FIG. 6**

**FIG. 7**

EP 4 364 711 A1

```
                        ┌──────────┐
                        │  Start   │
                        └──────────┘
                             │
                             ▼                        ╭─ 810
    ┌─────────────────────────────────────────────────────┐
    │      Determine target exercise mode of wearable device │
    └─────────────────────────────────────────────────────┘
                             │
                             ▼                        ╭─ 820
    ┌─────────────────────────────────────────────────────┐
    │ Determine value of first control parameter based on target exercise mode │
    └─────────────────────────────────────────────────────┘
                             │
                             ▼                        ╭─ 830
    ┌─────────────────────────────────────────────────────┐
    │        Generate sensing data based on raw sensing data and value of │
    │                   first control parameter             │
    └─────────────────────────────────────────────────────┘
                             │
                             ▼                        ╭─ 840
    ┌─────────────────────────────────────────────────────┐
    │ Generate target state factor corresponding to target type of target exercise │
    │                 mode based on sensing data            │
    └─────────────────────────────────────────────────────┘
                             │
                             ▼                        ╭─ 850
    ┌─────────────────────────────────────────────────────┐
    │ Determine value of second control parameter based on target exercise mode │
    └─────────────────────────────────────────────────────┘
                             │
                             ▼                        ╭─ 860
    ┌─────────────────────────────────────────────────────┐
    │        Determine torque value corresponding to target type of │
    │    target exercise mode based on target state factor and value of │
    │                   second control parameter            │
    └─────────────────────────────────────────────────────┘
                             │
                             ▼                        ╭─ 870
    ┌─────────────────────────────────────────────────────┐
    │        Control wearable device based on torque value   │
    └─────────────────────────────────────────────────────┘
                             │
                             ▼
                        ┌──────────┐
                        │   End    │
                        └──────────┘
```

# FIG. 8

Start

Receive information on one or more exercise modes from user — 910

Generate exercise program based on exercise modes — 920

To operation 810

**FIG. 9**

FIG. 10

EP 4 364 711 A1

1100

| Exercise program | | |
|---|---|---|
| **Exercise mode** | **Time** | **Intensity** |
| Knee up | 5 min  + − | Second step  + − |
| Squat | 5 min  + − | Third step  + − |
| Interval walking | 20 min  + − | Second step  + − |

## FIG. 11

1200

1202

5.0 / 5.0 Nm — 1204

10W / 10W — 1206

| | | |
|---|---|---|
| Intensity (gain) | + − | 5 |
| Timing (delay) | + − | 0.25 |
| Sensitivity | + − | 0.05 |
| Offset R | + − | 0 |
| Offset L | + − | 0 |

1210

| Step | | Non-step | ✓ |
|---|---|---|---|

1220

1230 — Good morning | Save | Del

**FIG. 12**

From operation 830

Generate feedback information on execution of target exercise mode
based on sensing data /1310

Output feedback information /1320

End

## FIG. 13

**FIG. 14A**

FIG. 14B

FIG. 14C

FIG. 15A

**FIG. 15B**

FIG. 15C

Start

810

1610

Obtain sensing data

1620

Determine target exercise mode corresponding to sensing data
from among exercise modes

To operation 810

# FIG. 16

1710

Start

810

Determine target exercise mode of wearable device

840

Generate target state factor corresponding to target type of
target exercise mode based on sensing data

850

Determine value of control parameter for magnitude of torque to be
output by wearable device based on target exercise mode

860

Determine torque value corresponding to target type based on
target state factor and value of control parameter

870

Control wearable device based on torque value

End

**FIG. 17A**

1720

```
            ┌─────────────┐
            │    Start    │
            └──────┬──────┘
                   │                                      810
     ┌─────────────▼──────────────────────────────────┐
     │  Determine target exercise mode of wearable device │
     └─────────────┬──────────────────────────────────┘
                   │                                      840
     ┌─────────────▼──────────────────────────────────┐
     │  Generate target state factor corresponding to    │
     │  target type of target exercise mode based on      │
     │  sensing data                                      │
     └─────────────┬──────────────────────────────────┘
                   │                                      850
     ┌─────────────▼──────────────────────────────────┐
     │  Determine value of control parameter for output   │
     │  timing of torque to be output by wearable device  │
     │  and value of control parameter for magnitude of   │
     │  torque, based on target exercise mode             │
     └─────────────┬──────────────────────────────────┘
                   │                                      860
     ┌─────────────▼──────────────────────────────────┐
     │  Determine torque value corresponding to target    │
     │  type based on target state factor and values of   │
     │  control parameters                                │
     └─────────────┬──────────────────────────────────┘
                   │                                      870
     ┌─────────────▼──────────────────────────────────┐
     │  Control wearable device based on torque value     │
     └─────────────┬──────────────────────────────────┘
                   │
            ┌──────▼──────┐
            │     End     │
            └─────────────┘
```

# FIG. 17B

1730

```
                    ┌──────────┐
                    │  Start   │
                    └─────┬────┘
                          │                         810
        ┌─────────────────▼─────────────────────────────┐
        │ Determine target exercise mode of wearable device │
        └─────────────────┬─────────────────────────────┘
                          │                         820
        ┌─────────────────▼─────────────────────────────┐
        │ Determine value of first control parameter for    │
        │ adjusting sensitivity of raw sensing data         │
        └─────────────────┬─────────────────────────────┘
                          │                         830
        ┌─────────────────▼─────────────────────────────┐
        │ Generate sensing data based on raw sensing data   │
        │ and value of first control parameter              │
        └─────────────────┬─────────────────────────────┘
                          │                         840
        ┌─────────────────▼─────────────────────────────┐
        │ Generate target state factor corresponding to     │
        │ target type of target exercise mode based on      │
        │ sensing data                                      │
        └─────────────────┬─────────────────────────────┘
                          │                         850
        ┌─────────────────▼─────────────────────────────┐
        │ Determine value of second control parameter for   │
        │ magnitude of torque to be output by wearable      │
        │ device based on target exercise mode              │
        └─────────────────┬─────────────────────────────┘
                          │                         860
        ┌─────────────────▼─────────────────────────────┐
        │ Determine torque value corresponding to target    │
        │ type based on target state factor and value of    │
        │ second control parameter                          │
        └─────────────────┬─────────────────────────────┘
                          │                         870
        ┌─────────────────▼─────────────────────────────┐
        │ Control wearable device based on torque value     │
        └─────────────────┬─────────────────────────────┘
                          │
                    ┌─────▼────┐
                    │   End    │
                    └──────────┘
```

# FIG. 17C

1740

```
                            ┌──────────┐
                            │  Start   │
                            └──────────┘
                                 │
                                 ▼
┌───────────────────────────────────────────────────────────┐  810
│        Determine target exercise mode of wearable device   │
└───────────────────────────────────────────────────────────┘
                                 │
                                 ▼
┌───────────────────────────────────────────────────────────┐  820
│  Determine value of first control parameter indicating     │
│  offset angle between joint angles of raw sensing data     │
└───────────────────────────────────────────────────────────┘
                                 │
                                 ▼
┌───────────────────────────────────────────────────────────┐  830
│   Generate sensing data based on raw sensing data and      │
│   value of first control parameter                         │
└───────────────────────────────────────────────────────────┘
                                 │
                                 ▼
┌───────────────────────────────────────────────────────────┐  840
│   Generate target state factor corresponding to target     │
│   type of target exercise mode based on sensing data       │
└───────────────────────────────────────────────────────────┘
                                 │
                                 ▼
┌───────────────────────────────────────────────────────────┐  850
│  Determine value of second control parameter for magnitude │
│  of torque to be output by wearable device based on        │
│  target exercise mode                                      │
└───────────────────────────────────────────────────────────┘
                                 │
                                 ▼
┌───────────────────────────────────────────────────────────┐  860
│   Determine torque value corresponding to target type      │
│   based on target state factor and value of second         │
│   control parameter                                        │
└───────────────────────────────────────────────────────────┘
                                 │
                                 ▼
┌───────────────────────────────────────────────────────────┐  870
│        Control wearable device based on torque value       │
└───────────────────────────────────────────────────────────┘
                                 │
                                 ▼
                            ┌──────────┐
                            │   End    │
                            └──────────┘
```

**FIG. 17D**

1750

Start

810

Determine target exercise mode of wearable device

820

Determine value of first control parameter indicating offset angle between joint angles of raw sensing data

830

Generate sensing data based on raw sensing data and value of first control parameter

840

Generate target state factor corresponding to target type of target exercise mode based on sensing data

850

Determine value of second control parameter including value of control parameter for output timing of torque to be output by wearable device and value of control parameter for magnitude of torque, based on target exercise mode

860

Determine torque value corresponding to target type based on target state factor and value of second control parameter

870

Control wearable device based on torque value

End

# FIG. 17E

1760

Start

810

Determine target exercise mode of wearable device

820

Determine value of first control parameter including value of
control parameter for adjusting sensitivity of raw sensing data

830

Generate sensing data based on raw sensing data and value of
first control parameter

840

Generate target state factor corresponding to target type of
target exercise mode based on sensing data

850

Determine value of second control parameter
including value of control parameter for output timing of torque to be
output by wearable device and value of control parameter for
magnitude of torque, based on target exercise mode

860

Determine torque value corresponding to target type based on
target state factor and value of second control parameter

870

Control wearable device based on torque value

End

# FIG. 17F

1770

```
                    ┌─────────┐
                    │  Start  │
                    └────┬────┘
                         │
                         ▼                                810
┌──────────────────────────────────────────────────────────┐
│         Determine target exercise mode of wearable device │
└──────────────────────────┬───────────────────────────────┘
                           │
                           ▼                              820
┌──────────────────────────────────────────────────────────┐
│  Determine value of first control parameter including      │
│  value of control parameter for adjusting sensitivity of   │
│  raw sensing data and value of control parameter           │
│  indicating offset angle between joint angles of           │
│  raw sensing data                                          │
└──────────────────────────┬───────────────────────────────┘
                           │
                           ▼                              830
┌──────────────────────────────────────────────────────────┐
│      Generate sensing data based on raw sensing data       │
│         and value of first control parameter               │
└──────────────────────────┬───────────────────────────────┘
                           │
                           ▼                              840
┌──────────────────────────────────────────────────────────┐
│   Generate target state factor corresponding to target     │
│   type of target exercise mode based on sensing data       │
└──────────────────────────┬───────────────────────────────┘
                           │
                           ▼                              850
┌──────────────────────────────────────────────────────────┐
│  Determine value of second control parameter including     │
│  value of control parameter for magnitude of torque to be  │
│  output by wearable device, based on target exercise mode  │
└──────────────────────────┬───────────────────────────────┘
                           │
                           ▼                              860
┌──────────────────────────────────────────────────────────┐
│  Determine torque value corresponding to target type       │
│  based on target state factor and value of second          │
│  control parameter                                         │
└──────────────────────────┬───────────────────────────────┘
                           │
                           ▼                              870
┌──────────────────────────────────────────────────────────┐
│        Control wearable device based on torque value       │
└──────────────────────────┬───────────────────────────────┘
                           │
                           ▼
                    ┌─────────┐
                    │   End   │
                    └─────────┘
```

# FIG. 17G

## INTERNATIONAL SEARCH REPORT

| International application No. |
|---|
| **PCT/KR2022/021594** |

### A. CLASSIFICATION OF SUBJECT MATTER

**A61H 1/02**(2006.01)i; **B25J 9/00**(2006.01)i; **A61H 37/00**(2006.01)i

According to International Patent Classification (IPC) or to both national classification and IPC

### B. FIELDS SEARCHED

Minimum documentation searched (classification system followed by classification symbols)

A61H 1/02(2006.01); A61H 3/00(2006.01); A63B 21/00(2006.01); A63B 21/005(2006.01); B25J 9/00(2006.01); G05B 13/02(2006.01); G06F 3/041(2006.01); G06F 3/0488(2013.01); H04W 4/00(2009.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 외부 장치(external device), 통신 모듈(communication module), 프로세서 (processor), 타겟 운동 모드(target exercise mode), 제어 파라미터(control parameter), 센싱 데이터(sensing data), 타겟 상태 인자(target state factor), 토크(torque)

### C. DOCUMENTS CONSIDERED TO BE RELEVANT

| Category* | Citation of document, with indication, where appropriate, of the relevant passages | Relevant to claim No. |
|---|---|---|
| Y | KR 10-2021-0054086 A (SAMSUNG ELECTRONICS CO., LTD.) 13 May 2021 (2021-05-13)<br>See paragraphs [0040]-[0058]; and figure 3. | 1-15 |
| Y | KR 10-2010-0068716 A (SAMSUNG ELECTRONICS CO., LTD.) 24 June 2010 (2010-06-24)<br>See claim 1. | 1-15 |
| Y | KR 10-2019-0057566 A (EWHA UNIVERSITY - INDUSTRY COLLABORATION FOUNDATION) 29 May 2019 (2019-05-29)<br>See paragraphs [0052]-[0081]; claim 1; and figures 1-4. | 1-15 |
| A | JP 2006-204426 A (YOSHIYUKI, Yamaumi) 10 August 2006 (2006-08-10)<br>See paragraphs [0025]-[0115]; and figures 1-13. | 1-15 |
| A | US 2021-0121729 A1 (SAMSUNG ELECTRONICS CO., LTD.) 29 April 2021 (2021-04-29)<br>See paragraphs [0053]-[0082]; and figures 3-5. | 1-15 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **06 April 2023** | **07 April 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

| International application No. |
|---|
| **PCT/KR2022/021594** |

| Patent document cited in search report | | | Publication date (day/month/year) | Patent family member(s) | | | Publication date (day/month/year) |
|---|---|---|---|---|---|---|---|
| KR | 10-2021-0054086 | A | 13 May 2021 | CN | 112774115 | A | 11 May 2021 |
| | | | | EP | 3815666 | A1 | 05 May 2021 |
| | | | | US | 11590383 | B2 | 28 February 2023 |
| | | | | US | 2021-0128972 | A1 | 06 May 2021 |
| KR | 10-2010-0068716 | A | 24 June 2010 | KR | 10-1030398 | B1 | 20 April 2011 |
| | | | | US | 2010-0149130 | A1 | 17 June 2010 |
| KR | 10-2019-0057566 | A | 29 May 2019 | KR | 10-2009087 | B1 | 08 August 2019 |
| JP | 2006-204426 | A | 10 August 2006 | AT | 526000 | T | 15 October 2011 |
| | | | | CA | 2596346 | A1 | 03 August 2006 |
| | | | | CA | 2596346 | C | 29 June 2010 |
| | | | | CN | 101111211 | A | 23 January 2008 |
| | | | | CN | 101111211 | B | 19 January 2011 |
| | | | | DK | 1842518 | T3 | 05 December 2011 |
| | | | | DK | 2392305 | T3 | 24 June 2019 |
| | | | | EP | 1842518 | A1 | 10 October 2007 |
| | | | | EP | 1842518 | A4 | 20 January 2010 |
| | | | | EP | 1842518 | B1 | 28 September 2011 |
| | | | | EP | 2392305 | A1 | 07 December 2011 |
| | | | | EP | 2392305 | B1 | 03 April 2019 |
| | | | | JP | 4178187 | B2 | 12 November 2008 |
| | | | | KR | 10-0904937 | B1 | 29 June 2009 |
| | | | | KR | 10-2007-0092312 | A | 12 September 2007 |
| | | | | RU | 2007-132169 | A | 10 March 2009 |
| | | | | RU | 2364385 | C2 | 20 August 2009 |
| | | | | US | 2008-0161937 | A1 | 03 July 2008 |
| | | | | US | 2011-0004322 | A1 | 06 January 2011 |
| | | | | US | 2015-0150747 | A1 | 04 June 2015 |
| | | | | US | 7857774 | B2 | 28 December 2010 |
| | | | | US | 8932241 | B2 | 13 January 2015 |
| | | | | US | 9427373 | B2 | 30 August 2016 |
| | | | | WO | 2006-080134 | A1 | 03 August 2006 |
| US | 2021-0121729 | A1 | 29 April 2021 | KR | 10-2021-0050636 | A | 10 May 2021 |

Form PCT/ISA/210 (patent family annex) (July 2022)